# EUROPEAN PATENT APPLICATION

(11) **EP 3 276 354 A1**
(43) Date of publication of application: **31.01.2018**
(21) Application number: 16181994.1
(22) Date of filing: 29.07.2016
(51) Int. Cl.: G01N 33/68, A61K 49/08, A61K 49/14, G01N 33/84, B82Y 5/00, C07D 493/14

(54) **MOLECULAR SENSOR FOR NMR/MRI BASED ON ANALYTE-DEPENDENT SPECTRAL CHANGES OF TEMPORARILY ENCAPSULATED HYPERPOLARIZED 129XE**

(71) Applicant: Klinikum rechts der Isar der Technischen Universität München, 81675 München (DE); Forschungsverbund Berlin e.V., 12489 Berlin (DE)
(72) Inventor: Westmeyer, Gil Gregor, 80804 München (DE); Truong, Dong-Jiunn Jeffery, 85356 Freising (DE); Schröder, Leif, 10115 Berlin (DE); Rossella, Federica, 12047 Berlin (DE); Witte, Christopher, 10439 Berlin (DE)
(74) Representative: Engelhard, Markus

(57) **Abstract**

The present invention relates to a precursor of a molecular sensor for determining analyte concentrations and/or measuring analyte concentration changes comprising a host for an active nucleus, an NMR-modulating moiety and an interacting moiety, wherein said NMR-modulating moiety changes the resonance frequency or the chemical exchange saturation transfer (CEST) signal of the active nucleus-host complex, and wherein said interacting moiety specifically responds to an environmental parameter, to an analyte or to a target molecule that binds the analyte or said interacting moiety specifically binds to a target molecule in an analyte-dependent manner. The present invention further relates to a molecular sensor comprising an active nucleus and said precursor. The present invention further relates to a molecular sensor for determining analyte concentrations and/or measuring analyte concentration changes inside a cell, wherein moiety/ies of the sensor are expressed in said cells and then assembled inside said cell.

The present invention further relates to uses of the molecular sensors as well as to an *in vitro* method for determining metal concentration and/or measuring metal concentration changes and a method for diagnosing and/or monitoring treatment of diseases causing changes in metal concentrations.

## Description

The present invention relates to a precursor of a molecular sensor for determining analyte concentrations and/or measuring analyte concentration changes comprising a host for an active nucleus, an NMR-modulating moiety and an interacting moiety, wherein said NMR-modulating moiety changes the resonance frequency or the chemical exchange saturation transfer (CEST) signal of the active nucleus-host complex, and wherein said interacting moiety specifically responds to an environmental parameter, to an analyte or to a target molecule that binds the analyte or said interacting moiety specifically binds to a target molecule in an analyte-dependent manner. The present invention further relates to a molecular sensor comprising an active nucleus and said precursor. The present invention further relates to a molecular sensor for determining analyte concentrations and/or measuring analyte concentration changes inside a cell, wherein moiety/ies of the sensor are expressed in said cells and then assembled inside said cell.

The present invention further relates to uses of the molecular sensors as well as to *an in vitro* method for determining metal concentration and/or measuring metal concentration changes and a method for diagnosing and/or monitoring treatment of diseases causing changes in metal concentrations.

### BACKGROUND OF THE INVENTION

Metal ions are of fundamental importance for biological processes since they function as essential constituents of structural proteins and enzymes, or as signaling molecules and second messengers. The most prominent example of the last category is calcium, which is important in many signal-transduction cascades and also essential for the signaling in electrically excitable cells, in particular the electrochemical conversion at the presynaptic synapses of neurons mediated through voltage gated influx and intracellular release of calcium as a function of membrane potential. Organ(ism)s thus have to tightly control the concentration of metals and their spatiotemporal distribution as many factors including e.g. increased metabolic demand, malnutrition or contact with toxic concentrations of metals present for instance in environmental pollutants can cause deflections from the physiological range.

Synthetic metal sensors are available for optical readouts based on absorbance (Durham *et al.,* 1983) and fluorescence (Carter *et al.,* 2014). There also exist genetically encoded sensors for readout via fluorescence (Looger *et al.,* 2013) and bioluminescence (in conjunction with appropriate synthetic substrates). These photon-detected metal sensors however all suffer from the poor penetration of photons in biological tissue or in opaque and/or turbid samples. The penetration depth depends on the specific method and desired resolution; even optimal use of near-infrared illumination and independence from scattering as achieved by optacoustics however still limits the maximum penetration depth in biological tissues to a few centimeters (Ntziachristos *et al.,* 2010). For these photon-based imaging methods (the wavelengths being in the visible range) to measure deeper *structures in vivo,* invasive methods are necessary that e.g. surgically insert optical guides such as fiber bundles into tissue. For diagnostic measurements *of in vitro* or *ex-vivo* samples (such as biological fluids and tissue as well also non-medical samples such as waste water) photon-dependent methods are also severely limited in their performance by the samples' opacity and turbidity.

It would therefore be of great value for biomedical diagnostics as well as for applications in food and environmental testing if robust photon-independent detection of metals in opaque and turbid media and non-invasive *in vivo* imaging of their distribution in organ(ism)s could be achieved at sub-millimeter resolution.

One non-invasive method that fulfills these criteria is nuclear Magnetic Resonance Imaging (MRI) that routinely achieves whole-body coverage in humans. Metal-sensitive contrast agents for NMR and MRI have been developed previously. They fall into four categories according to their physical mechanism: T₂ relaxation agents, T₁ relaxation agents, chemical shift and CEST agents. However, these methods suffer from a lack of sensitivity, obviously one of the major obstacles for *in vivo* MRI in general, necessitating the use of higher micromolar if not millimolar concentrations of the contrast agent.

To improve MRI's sensitivity, several so-called hyperpolarization methods have been developed that can increase the polarization of nuclear spins and thus the available signal (Viale *et al.* 2009, Viale *et al.* 2010). In the case of the noble gases helium (³He) and xenon (¹²⁹Xe), hyperpolarization can be achieved via optical pumping.

Caged xenon has been described as MRI reporter or biosensor. See e.g. Klippel *et al.,* 2014; Schröder 2007; Rose *et al.,* 2014.

Klippel *et al.* (2014) describe MRI localization of cells labeled with caged xenon in a packed-bed bioreactor working under perfusion with hyperpolarized-xenon-saturated medium. Xenon hosts enable NMR/MRI experiments with switchable contrast and selectivity for cell-associated versus unbound cages.

Rose *et al.* (2014) describe a functional xenon NMR biosensor that can identify the cell surface protein CD 14 by targeted ¹²⁹Xe MRI. Cells expressing CD 14 can be spatially distinguished from control cells with incorporation of the xenon MRI readout unit, cryptophane-A. The recognition of CD 14 is accomplished via a CD14-specific antibody. Witte *et al.* (2015) describe Xenon-MRI on living cells with hyper-CEST biosensors for metabolically labelled glycans on the cell surface.

US 6,652,833 B2 describes a functionalized active-nucleus complex that selectively associates with a biological target species, wherein the functionalized active-nucleus complex comprises: a) an active nucleus and b) a cryptophane family member targeting carrier comprising: i) a first binding region having at least a minimal transient binding of said active-nucleus to form the functionalized active-nucleus complex that produces a detectable signal when the functionalized active-nucleus complex associates with the target species and ii) a second binding region, non-coextensive with said first binding region, that selectively associates with the target species.

Here as well as in other earlier presented sensors, the cryptophane family member or Xe host is always accessible for reversibly bound Xe atoms and thus provides a measurable CEST effect even under conditions where the sensor is dissociated from the target or not in an environment that has certain biochemical "target conditions" (such as temperature, viscosity, pH, a certain ion concentration, 02 level, etc.). The sensing capability of such earlier presented sensors therefore does not rely on changing the Xe access to the host.

Thus, there is a need for improved means and methods for analyte sensing *for in vitro and in vivo* applications.

### SUMMARY OF THE INVENTION

According to the present invention this object is solved by a precursor for a molecular sensor for determining analyte concentrations and/or measuring analyte concentration changes comprising
(a) a host for an active nucleus,
   preferably a cage-like molecule,
   wherein said host enables at least a transient binding of said active-nucleus that produces a detectable NMR signal when the sensor binds to the analyte,
(b) an NMR-modulating moiety,
   wherein said NMR-modulating moiety changes the resonance frequency or the chemical exchange saturation transfer (CEST) signal of the active nucleus-host complex,
   and
(c) an interaction moiety,
   wherein said interaction moiety specifically responds to an environment parameter (such as temperature or viscosity), an analyte or a target molecule that binds the analyte
   or said interaction moiety specifically binds to a target molecule in an analyte-dependent manner,
wherein the analyte is preferably a metal, more preferably calcium, or a protein.

According to the present invention this object is solved by a precursor for a molecular sensor for determining analyte concentrations and/or measuring analyte concentration changes
comprising
an active nucleus,
preferably xenon, more preferably hyperpolarized xenon,
and the precursor of the present invention,
wherein the analyte is preferably a metal, more preferably calcium, or a protein.

According to the present invention this object is solved by the use of a molecular sensor of the present invention for
- determining analyte concentrations and/or measuring analyte concentration changes,
- *in vitro, ex vivo and in vivo* measurements of spatiotemporal analyte distributions,
- *in vivo* imaging of analyte distributions in preclinical animal models and humans, in particular for diagnosing and/or monitoring treatment of diseases associated with changes in metal concentrations.

According to the present invention this object is solved by the molecular sensor of the present invention for use as a medicament.

According to the present invention this object is solved by the molecular sensor of the present invention for use in a method of diagnosing and/or monitoring treatment of diseases causing changes in metal concentrations.

According to the present invention this object is solved by an *in vitro* method for determining metal concentration and/or measuring metal concentration changes in a sample, comprising the steps of
(i) providing the precursor of a molecular sensor according to the present invention,
(ii) providing an active nucleus of said molecular sensor to the sample, preferably a spin-hyperpolarized nucleus,
(iii) performing nuclear magnetic resonance (NMR) spectroscopy and imaging (MRS, MRI, Hyper-CEST) in a sample,
   optionally performing further measurement(s) to detect the further sensor moiety(moieties),
(iv) determining metal concentrations or metal concentration changes of or in the sample.

According to the present invention this object is solved by a method for diagnosing and/or monitoring treatment of diseases causing changes in metal concentrations, comprising the steps of
(i) administering to the body of a patient or nun-human animal the precursor of a molecular sensor according to the present invention,
(ii) providing an active nucleus of said molecular sensor to the region or tissue of interest,
   preferably a spin-hyperpolarized nucleus,
(iii) *performing in vivo* nuclear magnetic resonance (NMR) spectroscopy and imaging (MRS, MRI, Hyper-CEST),
   optionally performing further measurement(s) to detect the further sensor moiety(moieties),
(iv) determining metal concentrations or metal concentration changes of or in the body of said patient or nun-human animal.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "5 to 40 amino acids" should be interpreted to include not only the explicitly recited values of 5 to 40, but also include individual values and sub-ranges within the indicated range. Thus, included in this numerical range are individual values such as 5, 6, 7, 8, 9, 10... 38, 39, 40 and sub-ranges such as from 10 to 30, 15 to 25, from 20 to 35, etc. This same principle applies to ranges reciting only one numerical value, such as "at least 5 amino acids". Furthermore, such an interpretation should apply regardless of the breadth of the range or the characteristics being described.

### Molecular analyte sensors and their prescursors

The present invention provides a precursor to a molecular sensor for determining analyte concentrations and/or measuring analyte concentration changes.

The present invention provides a molecular sensor for determining analyte concentrations and/or measuring analyte concentration changes.

The analyte is preferably a metal (or metal ion), more preferably calcium (or calcium ions). The analyte can be any ion/atomic/molecular structure that induces a conformational change that has impact on the NMR-modulating moiety (b) and interaction moiety (c).

The analyte can be a protein.

Said precursor to a molecular sensor comprises
(a) a host for an active nucleus,
(b) an NMR-modulating moiety, and
(c) an interaction moiety.

Said molecular sensor comprises
an active nucleus,
and
the precursor, which comprises
(a) a host for the active nucleus,
(b) an NMR-modulating moiety, and
(c) an interaction moiety.

Said precursor can comprise in addition to (a) to (c) or said molecular sensor can comprise in addition to (a) to (c) and the active nucleus:
(d) optionally, further sensor moiety/ies,
(e) optionally, solubilizing and/or biodistribution moiety/ies,
(f) optionally, further interacting moiety/ies.

In a preferred embodiment, the molecular sensor is assembled inside a (target) cell or tissue.

### Active nucleus

The active nucleus is preferably xenon ¹²⁹Xe, more preferably hyperpolarized xenon.
The active nucleus can be any other nucleus participating in reversible exchange and CEST detection, such as ¹³C, ¹⁵N, ¹⁹F, ²⁹Si, ³¹P, ⁸⁹Y.

### (a) Host for the active nucleus

Said host enables at least a transient binding of said active-nucleus that produces a detectable NMR signal when the sensor binds to the analyte.

The host is preferably a cage-like molecule.

The host depends on the active nucleus to be hosted.

The active nucleus and the host form an active nucleus-host complex.
In said active nucleus-host complex the nucleus changes its NMR resonance condition upon binding to the host.

In a preferred embodiment, the active nucleus is xenon and the host is a xenon host.

A xenon host is preferably selected from a cryptophane, cucurbit[n]uriles, pillar[n]arenes, or self-assembling metal-organic cages,
including genetically encoded compartments consisting of proteins, peptides, DNA and RNA. See e.g. Lowery *et al.* (2004) that describe a conformation-sensitive xenon-binding cavity in the ribose-binding protein. See e.g. Rubin *et al.* (2002) that describe Xenon-binding sites in Proteins which can be detected by ¹²⁹Xe NMR spectroscopy.

Cryptophanes are a class of organic supramolecular compounds for molecular encapsulation and recognition. Cryptophane cages are formed by two cup-shaped [1.1.1]-orthocyclophane units connected by three bridges (denoted *Y*). There are also choices of the peripheral substitutes *R1* and *R2* attached to the aromatic rings of the units. Most cryptophanes exhibit two diastereomeric forms (syn and anti), distinguished by their symmetry type. This general scheme offers a variety of choices *(Y, R1, R2,* and symmetry type) by which the shape, the volume, and the chemical properties of the generally hydrophobic pocket inside the cage can be modified, making cryptophanes suitable for encapsulating many types of small molecules and even chemical reactions.

Cryptophanes form with xenon host-guest complexes that are of reversible nature, i.e. the noble gas is in continuous exchange between its bound state in the host and the unbound state in solution.

Cucurbiturils are macrocyclic molecules made of glycoluril (=C₄H₂N₄O₂=) monomers linked by methylene bridges (-CH₂-). The oxygen atoms are located along the edges of the band and are tilted inwards, forming a partly enclosed cavity. Cucurbiturils are commonly written as cucurbit[*n*]uril, *where n* is the number of glycoluril units (and *n* can be e.g. 5, 6, 7, 8, and 10). Two common abbreviations are CB[*n*], or simply CB*n*. Cucurbiturils are efficient host molecules in molecular recognition and have a particularly high affinity for positively charged or cationic compounds. They have been identified as efficient Hyper-CEST agents (Kunth *et al.* 2015).

Pillararenes or pillar[n]arenes are macrocycles composed of hydroquinone units (n = 5 to 10) linked lby methylene bridges in the para-position. They are structurally similar to the cucurbiturils and have been studied as Xe hosts (Aldiri *et al,* 2013).

### (b) NMR-modulating moiety

The NMR-modulating moiety changes the resonance frequency or the chemical exchange saturation transfer (CEST) signal of the active nucleus-host complex.

This modulation can be influenced by an interaction of the interacting moiety.

### (c) Interaction moiety

The interaction moiety mediates interactions with environmental parameters, target molecules, or analytes.

The interaction moiety specifically responds to an environmental parameter, an analyte or a target molecule that binds the analyte,
or said interaction moiety specifically binds to a target molecule in an analyte-dependent manner.

Environmental parameters can be temperature or viscosity.

In a preferred embodiment, the NMR-modulating moiety (b) decreases the CEST signal of the active nucleus-host complex.
More preferably, the NMR-modulating moiety (b) and the interaction moiety (c) (reversibly) suppress the CEST signal from the otherwise accessible host by a specific conformation of the NMR-modulating moiety (b) in the host vicinity, and the interaction with a target molecule via the interaction moiety (c) in an analyte-dependent manner unsuppresses the CEST signal/effect.

"Chemical exchange saturation transfer (CEST)" imaging is a relatively new MRI contrast approach in which exogenous or endogenous compounds containing either exchangeable protons or exchangeable molecules are selectively saturated and, after transfer of this saturation, detected indirectly through the water signal with enhanced sensitivity. In CEST MRI, transfer of magnetization is studied from a dilute to an abundant pool. CEST imaging requires sufficiently slow exchange on the MR time scale to allow selective irradiation of the protons of interest. As a consequence, magnetic labeling is not limited to radio-frequency saturation but can be expanded with slower frequency-selective approaches such as inversion and frequency labeling.

The "hyper-CEST effect" as used herein refers to the use of spin-hyperpolarized nuclei as the exchanging species.

The present invention uses an "analyte-triggered CEST effect" which refers to a change in the (hyper-)CEST performance, i.e., a modulation of the exchange properties in the reversible binding of the nuclei, induced by the interaction of moiety (c) with the analyte and mediated by moiety (b).

The molecular sensors of the present invention, preferably the Xenon-based sensors, operate by a reversible (un)suppression of a HyperCEST effect.

The interaction moiety (c) specifically binds to the analyte or to a target molecule that binds the analyte.
The interaction moiety (c) can specifically bind to a target molecule in an analyte-dependent manner.

The interaction moiety (c) can be or comprise a protein, polypeptide or peptide, such as
an antibody or antibody fragment,
a domain or part of a protein,
which are each specific to the analyte or to a target molecule that binds the analyte or which each specifically bind to a target molecule in an analyte-dependent manner.

The analyte is preferably a metal (or metal ion), more preferably calcium (or calcium ions).

In one embodiment, the NMR-modulating moiety (b) and the interaction moiety (c) are attached to each other or form a "*joint moiety".*

Thereby, the NMR-modulating moiety (b) and the interaction moiety (c) are preferably comprised in or consist of a protein, a polypeptide or a peptide, such as
an antibody or antibody fragment,
a domain or part of a protein,
which are each specific to the analyte or to a target molecule that binds the analyte or which each specifically bind to a target molecule in an analyte-dependent manner.

In an embodiment, where the analyte is calcium, and where the NMR-modulating moiety (b) and the interaction moiety (c) are attached to each other (in a "joint moiety"), the NMR-modulating moiety (b) and the interaction moiety (c) comprise or consist of a peptide selected from
RS20,
M13,
calcineurin A,
or another CaM-binding peptide or protein.

RS20 is a synthetic peptide derived from the calmodulin-binding region of smooth muscle myosin light chain kinase (smMLCK).
SEQ ID NO. 1
   RRKWQKTGHAVRAIGRLSSS

M13 is a synthetic peptide. Its sequence is the same as the calmodulin-binding domain of skeletal muscle myosin light chain kinase (skMLCK) (residues 577-602). Calmodulin can bind the M13 peptide without the presence of the entire protein.
SEQ ID NO. 2
   KRRWKKNFIAVSAANRFKKISSSGAL

Calcineurin A is the calmodulin-binding catalytic subunit of calcineurin, which is a calcium and calmodulin dependent serine/threonine protein phosphatase (also known as protein phosphatase 3, and calcium-dependent serine-threonine phosphatase).
SEQ ID NO. 3
   ARKEVIRNKIRAIGKMARVFSVLR

Calmodulin (CaM) (an abbreviation for calcium-modulated protein) is a multifunctional intermediate calcium-binding messenger protein expressed in all eukaryotic cells. It is an intracellular target of the secondary messenger Ca²⁺, and the binding of Ca²⁺ is required for the activation of Calmodulin. Once bound to Ca²⁺, Calmodulin acts as part of a calcium signal transduction pathway by modifying its interactions with various target proteins, such as myosin light chain kinase (MLCK), calmodulin-dependent kinases, protein phosphatase calcineurin, phosphodiesterase, nitric oxide synthase, Ca²⁺-ATPase pumps, as well as cytoskeletal structural proteins.
For example, homo sapiens CaM SEQ ID NO. 4

Examples for said other CaM-binding peptides or proteins are e.g. described in Barnes and Gomes (1995), Ashfar et al. (1994), Vetter and Leclerc (2003), Erickson-Viitanen and DeGrado (1987).

CaM-kinase II (CAMK2) is a prominent kinase in the central nervous system that may function in long-term potentiation and neurotransmitter release. Member of the NMDAR signaling complex in excitatory synapses it may regulate NMDAR-dependent potentiation of the AMPAR and synaptic plasticity.
SEQ ID NO. 5

MATITCTRFTEEYQLFEELGKGAFSVVRRCVKVLAGQEYAAKIINTKKLSARDHQKLERE
ARICRLLKHPNIVRLHDSISEEGHHYLIFDLVTGGELFEDIVAREYYSEADASHCIQQIL
EAVLHCHQMGVVHRDLKPENLLLASKLKGAAVKLADFGLAIEVEGEQQAWFGFAGTPGYL
SPEVLRKDPYGKPVDLWACGVILYILLVGYPPFWDEDQHRLYQQIKAGAYDFPSPEWDTV
TPEAKDLINKMLTINPSKRITAAEALKHPWISHRSTVASCMHRQETVDCLKKFNARRKLK
GAILTTMLATRNFSGGKSGGNKKNDGVKESSESTNTTIEDEDTKVRKQEIIKVTEQLIEA
ISNGDFESYTKMCDPGMTAFEPEALGNLVEGLDFHRFYFENLWSRNSKPVHTTILNPHIH
LMGDESACIAYIRITQYLDAGGIPRTAQSEETRVWHRRDGKWQIVHFHRSGAPSVLPH

Troponin (Tn) is the central regulatory protein of striated muscle contraction. Tn consists of three components: Tn-I which is the inhibitor of actomyosin ATPase, Tn-T which contains the binding site for tropomyosin and Tn-C. The binding of calcium to Tn-C abolishes the inhibitory action of Tn on actin filaments.
For example, homo sapiens Troponin SEQ ID NO. 6

MDDIYKAAVEQLTEEQKNEFKAAFDIFVLGAEDGCISTKELGKVMRMLGQNPTPEELQEM

IDEVDEDGSGTVDFDEFLVMMVRCMKDDSKGKSEEELSDLFRMFDKNADGYIDLDELKIM

LQATGETITEDDIEELMKDGDKNNDGRIDYDEFLEFMKGVE

The sensitivity of the molecular sensor of the present invention, such as a calcium sensor, can be changed/tuned by using variants of the target molecule or protein, such as calmodulin, with higher or lower affinities for the analyte (such as calcium).

The sensitivity of the molecular sensor can be tuned by using different target molecules or proteins that exhibit lower affinity for the analyte, such as proteins interacting with calcium at higher concentrations present in *e.g.* synaptic vesicles (*e.g.* C2A domain containing, lipid-binding proteins). This allows for tuning the sensor to extracellular calcium concentrations, thus avoiding the need for cellular delivery of the agent.

In one embodiment, the host (a), the NMR-modulating moiety (b) and the interaction moiety (c) are attached or connected to each other,
such as
via an amino acid side chain of the peptide, e.g. the side chain of a lysine,
via a linker or tether.

In a preferred embodiment, the NMR-modulating moiety (b) and the interaction moiety (c) comprises or consists of/is the peptide RS20 or the peptide M13 that specifically binds to an EF hand protein, such as calmodulin, in a calcium-dependent manner.

Preferably, the NMR-modulating moiety (b) and the interaction moiety (c), when attached/connected to each other/ "joint moiety") comprises or consists of the amino acid sequence selected from SEQ ID NOs. 1 to 3.

In a preferred embodiment the NMR-modulating and binding moiety (c) comprises or consists of the amino acid sequence of SEQ ID NO. 1 [RS20] or SEQ ID NO. 2 [M13] and is attached/linked via lysine side chains to the host.

The peptides can be linked to further sensor moiety/ies (as described below); such as fluorescein ("5-Fluo-M"). The host is cryptophane A ("Cr-A".) The maleimide-Fluorescein can be replaced by a maleimide capping group ("NEM").
Cr-A@K1_RS20_Fluorescein: SEQ ID NO. 7
   GRR-K(Cr-A)-WQKTGHAVRAIGRLSSS-C(5-Fluo-M)-amid
Cr-A@**K3_**RS20_Fluorescein: SEQ ID NO. 8
   GRRKWQK(Cr-A)-TGHAVRAIGRLSSS-C(5-Fluo-M)-amid
Cr-A@**K1**_M13_Fluorescein: SEQ ID NO. 9
   G-**K**(Cr-A)-RRWKKNFIAVSAANRFKKISSSGAL-C(5-Fluo-M)-amid
Cr-A@**K1**_RS20_NEM: SEQ ID NO. 10
   GRR-**K**(Cr-A)-WQKTGHAVRAIGRLSSS-C(NEM)-amid
Cr-A@**K1**_M13_NEM: SEQ ID NO. 11
   G-K(Cr-A)-RRWKKNFIAV SAANRFKKIS S S GAL-C(5-Fluo-M)-amid

In one embodiment, wherein the NMR-modulating moiety (b) and the interaction moiety (c) are attached to each other or form a joint moiety, preferably the NMR-modulating moiety (b) and the interaction moiety (c) are comprised in or consist of a linker,
such as polyethylene glycol (PEG).

In a preferred embodiment, the NMR-modulating moiety (b) and the interaction moiety (c) are attached to each other and comprise or consist of a peptide as defined herein, decrease the CEST signal of the active nucleus/ nucleus-host complex in aqueous solution and in absence of other compounds.
More preferably the CEST signal from the otherwise accessible host is suppressed by a specific conformation of the peptide in the host vicinity.

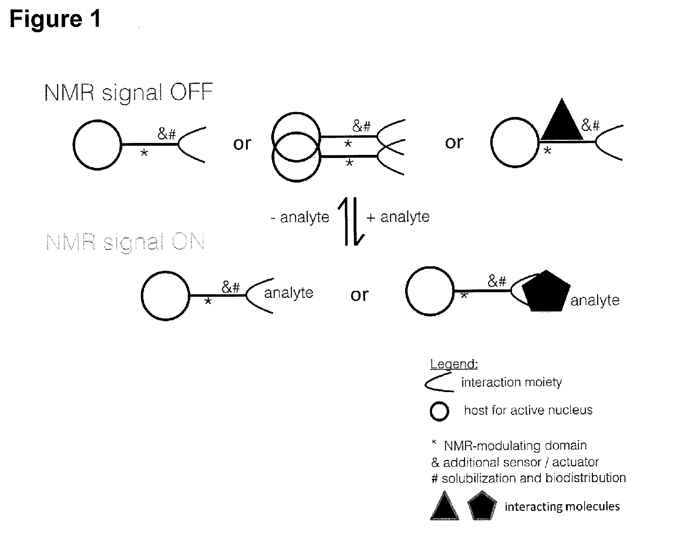

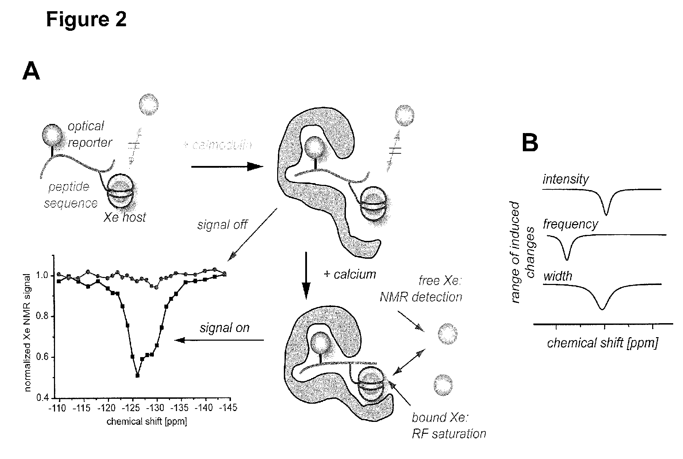

In the absence of the analyte (such as calcium ions) the saturation transfer signal from the cryptophane-encapsulated hyperpolarized ¹²⁹Xenon is suppressed by the peptide, with some contribution of the C-terminal modification (e.g. the further sensor moiety, such as fluorescein). If the analyte is added, the peptide binds tightly with the protein calmodulin and undergoes a conformational change, thereby turning on (unsuppressing) the saturation transfer signal in a calcium-dependent manner. The analyte-dependent NMR signal activation of this preferred embodiment thus occurs as schematized in Figures 1 and 2.

### Intracellular assembly of the molecular sensor inside the cell

As discussed above, in a preferred embodiment the molecular sensor for determining analyte concentrations and/or measuring analyte concentration changes in a cell / in a target cell, is assembled inside said cell or target cell or said tissue.

This can be realized by delivering the host for the active nucleus functionalized with respective moieties across the cellular membrane where it specifically bioconjugates with the other moieties of the sensor that are genetically expressed.

In another embodiment all moieties of the sensor including the host for the active molecule are genetically expressed.

The components (a) to (c) and the active nucleus can be delivered to the (target) cells. Moieties (a), (b) and/or (c) can be expressed by said (target) cells.

In one embodiment, the complex of the active nucleus and its host (a) or the host (a) are e.g. used and delivered to the target cells. The NMR-modulating moiety (b) and/or the interaction moiety (c), optionally fused to the target molecule of the analyte, will be provided by the target cells, such as via intracellular expression.

For example, the xenon-binding host molecule (a) can be delivered across the cellular membrane to "find" a specific genetically expressed calcium-binding protein such as a calmodulin-peptide fusion.

This can be achieved via bioconjugation via inteins, specific amino acid sequences including artificial amino acids and known chemistry such as click chemistry or known ligand to protein interaction such as biotin to avidin, all of which are known to people informed with the basic knowledge available in the field.

### Further components

In one embodiment, the precursor or the molecular sensor of the present invention further comprises
(d) a further sensor moiety/moieties,
   and/or
(e) solubilizing and/or biodistribution moiety/ies.

Said further further sensor moiety/moieties (d) and/or said solubilizing and/or biodistribution moiety/ies (e) can be covalenty attached to the NMR-modulating moiety (b) and/or the interaction moiety (c).

Said further sensor moiety or moieties (d) can be
- a further active nucleus-host complex,
- contrast agent(s),
   such as optically detectable contrast agents, PET contrast agents, ultrasound contrast agents),
- chromophore(s) and/or fluorophore(s),
   such as environmentally sensitive fluorophores,
   e.g. temperature, viscosity, pH, redox, metal-sensitive fluorophore- or chromophore-based sensors, effects of electromagnetic or mechanical waves, particle, irradiation,
   e.g. temperature, viscosity, pH, redox, metal-sensitive fluorophore- or chromophore-based sensors sensitive to electromagnetic or mechanical waves, particle, irradiation,
   e.g. fluorescein, ATTO, bodipy, cyanine, photo sensitizers,
- MRI or PET agent(s) or chelated transition metal(s),
   such as gadolinium DPTA, macrocycles,
- actuator(s),
   such as photosensitizers, magnetic structures,
- nanostructures,
   such as a gas-filled microbubble, a paramagnetic nanoparticle or gold particle,
- absorber,
or combinations thereof.

Said further sensor moiety can serve as reference signal, e.g. for quantifying the precursor or the molecular sensor of the invention.

For example, a ratiometric readout can be achieved by addition of a second (Xe) host (*e.g.* cucurbit or cryptophane) whose environment does not change as a function of analyte (calcium) binding (or other of the above-mentioned stimuli) such that it can be used to normalize the concentration of the sensor.

Said further sensor moiety can be used for localization of the precursor or the molecular sensor of the invention, e.g. *in in vivo* applications.
For example, localization of the precursor or the sensor by an independent read-out can be built in via a fluorophore, an MRI agent such as a T_1 agent represented by gadolinium DPTA (or similar chelates of paramagnetic ions), or a radioactive probe for PET detection.

This multimodal readout is possible by the precise knowledge of the three-dimensional structure of a primary analyte or of a target molecule, such as calmodulin, that affords the extension of the peptidic scaffold out of the binding pocket; external modifications by the modifiers mentioned above can thus be realized without interfering with the calcium-mediated peptide binding mechanisms.

Said further sensor moiety or moieties (d) can also enhance the NMR signal suppression effect.

Said solubilizing and/or biodistribution moiety or moieties (e) preferably is/are for modifying the solubility and biodistribution of the active-nucleus host complex, e.g. cell permeability. Preferably, it improves or enables cellular uptake and/or enables delivery over barriers, such as the blood-brain-barrier.

Said solubilizing and/or biodistribution moiety (e) aids in the delivery of the sensor of the present invention to a desired target organ (including the brain), target tissue and target cells.

The solubilizing and/or biodistribution moiety (e) can comprise respective receptor components, such as transferrin receptor or insulin receptor, or further specific binding moieties, such as antibodies or antibody fragments, which are specific for the desired target organ, tissue, cells.

In one embodiment, the precursor or the molecular sensor comprises a further interacting moiety or moieties (f).

Preferably, said further interacting moieties (f) aid in the binding of the precursor or the molecular sensor to the target molecule *in in vivo* application or in cellular applications or in applications where the target molecules are genetically modified and provided by intracellular expression.

Examples for said further interacting moieties (f) are
Biotin/Avidin,
Split Inteins
   such as split Npu DnaE intein or split gp41-1 intein,
Isopeptide-linked protein/peptide pairs,
   such as SpyTag/SpyCatcher,
or
antiparallel homodimeric protein interfaces.

For example, the precursor or molecular sensor of the invention which is to be delivered into the target cells comprises a biotin. The target molecule comprises an (strep)avidin.

For example, specificity for genetically defined cellular populations can be achieved by genetically expressing a variant of the target molecule, such as a calcium-binding protein, e.g. calmodulin, that exhibits a specific affinity for the peptide-cage construct (i.e. the molecular sensor) being delivered to the cell. This can be achieved by *e.g.* using high-affinity interacting moieties, such as SpyTag/SpyCatcher, Biotin/Avidin or antiparallel homodimeric protein interfaces.

### Preferred example for a calcium sensor:

the analyte is calcium or calcium ions (Ca²⁺),
the active nucleus is ¹²⁹Xenon,
   preferably spin-hyperpolarized ¹²⁹Xenon,
the host (a) is a xenon host,
   preferably a cryptophane,
   more preferably cryptophane A,
the NMR-modulating moiety (b) and the interaction moiety (c) are attached to each other and comprise or consist of a peptide selected from RS20, calcineurin A or M13, or another CaM-binding peptide,
   preferably comprising or consisting of the amino acid sequence selected from SEQ ID NOs. 1 to 3,
   more preferably RS20 with SEQ ID NO. 1 or M13 with SEQ ID NO. 2,
the host (a) and the joined NMR-modulating moiety (b) and the interaction moiety (c) are covalently attached to each other,
   preferably via a lysine side chain of RS20 or M13,
   more preferably via the side chain of amino acid residue 4 (= lysine) of SEQ ID NO. 1;
the target molecule is an EF hand protein,
   preferably calmodulin,
and the joined NMR-modulating moiety (b) and the interaction moiety (c) binds to calmodulin in a calcium-dependent manner,
the host (a) and the joined NMR-modulating moiety (b) and the interaction moiety (c) comprises a further sensor moiety (e),
preferably a chromophore, a fluorophore, an absorber, a macrocycle (for MRI, PET), or nanostructure, such as a gas-filled microbubble, a paramagnetic nanoparticle or gold particle,
more preferably a fluorophore, such as fluorescein.

The respective precursor of the calcium sensor comprises the above components without the active nucleus ¹²⁹Xenon, preferably spin-hyperpolarized ¹²⁹Xenon.

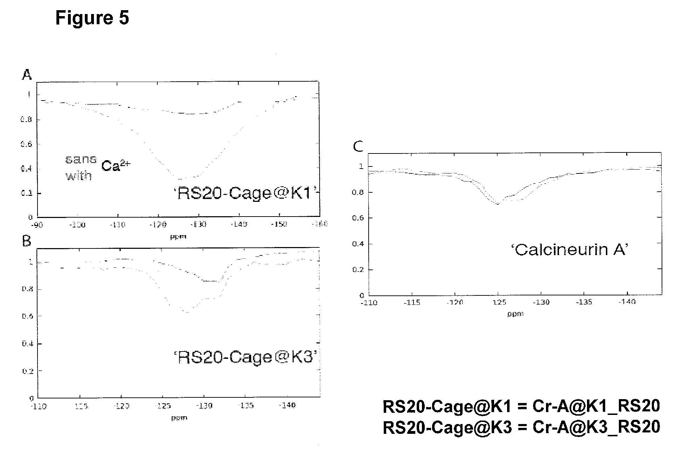

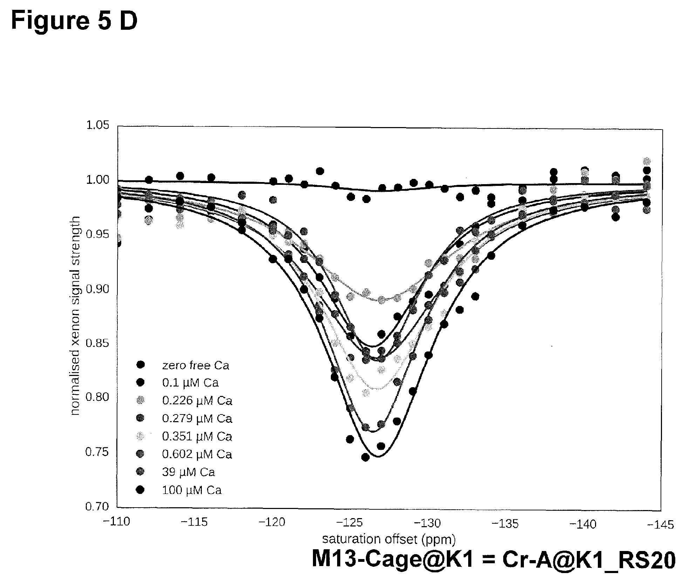

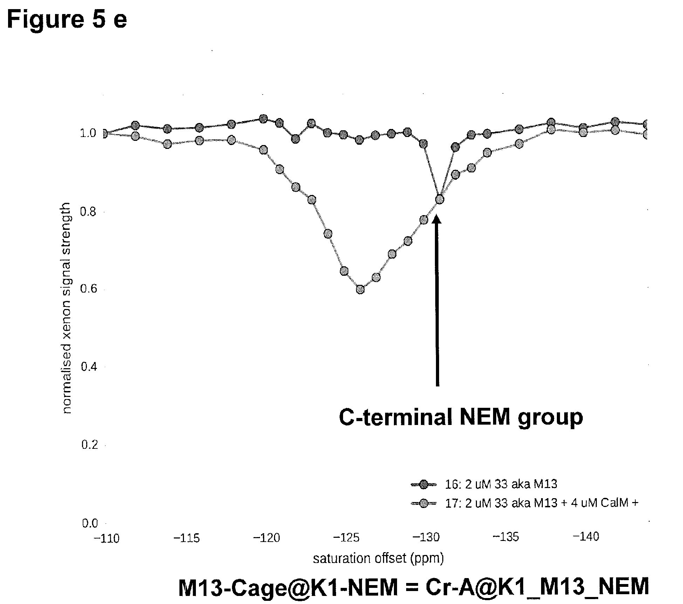

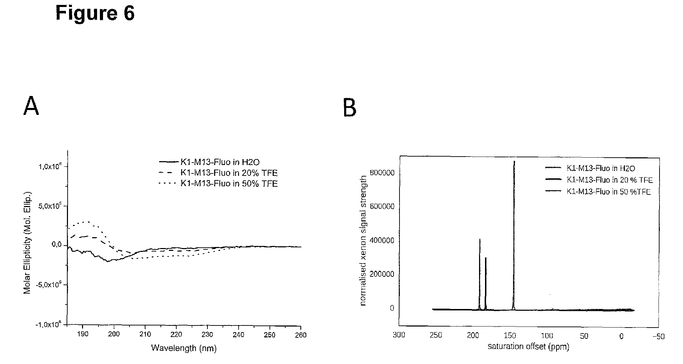

A particularly advantageous feature of a preferred embodiment of the sensor is a complete suppression of the CEST signal in the absence of calcium. As demonstrated in Figures 5 and 6, this previously unknown 'analyte-triggered turn on Hyper-CEST effect' depends on the specific choice of the peptide sequence and position of the cryptophane cage. Specifically, just shifting the cryptophane cage three amino acids more to the center of the RS20 peptide ("RS20-cage@K2") diminishes the CEST suppression and the amplitude of the signal change as compared with the sensor peptide ("RS20-cage@K1"). In addition, a C-terminal modification of the peptide with a fluorescein has a stronger CEST-suppression effect than a smaller moiety at the same position.

### Imaging and medical uses of the molecular sensor

The present invention provides the use of the molecular sensor of the present invention for
- determining analyte concentrations and/or measuring analyte concentration changes,
- *in vitro, ex vivo* and *in vivo* measurements of spatiotemporal analyte distributions,
- *in vivo* imaging of analyte distributions in preclinical animal models and humans, in particular for diagnosing and/or monitoring treatment of diseases associated with changes in metal concentrations.

The analyte is preferably a metal (or metal ion), more preferably calcium (or calcium ions).

The use according to the invention preferably comprises nuclear magnetic resonance (NMR) spectroscopy and imaging (MRS, MRI, Hyper-CEST).

The use according to the invention can comprise determining further analyte(s), such as further metal(s). In such an embodiment, the sensor of the present invention comprises further sensor moiety/moieties (d) that may e.g. operate via a different contrast agent property such as a change in the fluorescence or photoacoustic signal.

For example, the molecular sensor of the present invention can be used for sensing other external stimuli, such as the presence of other metals, and changes in environmental parameters, such as, temperature, viscosity or effects from electromagnetic waves, mechanical waves, or particles irradiation by using respective protein-peptide pairs (as the NMR-modulating and interaction moiety (b), (c) and optionally solubilizing and/or biodistribution moiety (e)).

The use according to the invention optionally comprises multimodal detection of further sensor moiety/moieties, preferably as defined herein above, via absorbance/transmission, reflection, fluorescence or optoacoustic or ultrasound measurements and imaging.

As used herein "multimodal detection" refers to the combined detection of the sensor of the present invention via a detection method that is not be based on the NMR, such as PET, ultrasound or photon-dependent methods.

In one embodiment, the magnetic resonance readout can be combined with photon-dependent imaging via transmission, fluorescence, or optoacoustic imaging techniques detecting a chromophore inserted.

As used herein "optoacoustic or photoacoustic" measurements or imaging refer to the detection of the mechanical waves generated by the optoacoustic, or photoacoustic effect (Ntziachristos *et al.,* 2010)

As used herein "fluorescent or absorption" measurements or imaging refer to the detection of fluorescent and/or absorbed photons for point measurements or spatiotemporally resolved measurements/imaging.

The use according to the invention preferably comprises
- *- ex vivo* (pre)clinical imaging of tissues and bodily fluids,
   such as blood, urine, lymph or lymphatic drainage, cerebrospinal fluid, stool/feces, semen, saliva or mucous fluids, or a cell culture sample, such as derived from the human or non-human animal body, *ex vivo* tissue, cell culture;
- *in vitro* measurement and imaging in biomedical or environmental samples,
   such as in cell and tissue cultures, metal screening in e.g. water, air, soil, plants, food.

The administration of the hyperpolarized ¹²⁹Xenon can be achieved for *in vitro* applications via bubbling/dispersing it into solution or via administration through the breathing air/Xe mixture or injection of a Xe-saturated carrier solution such as saline solution, blood plasma etc. for preclinical *in vivo* studies.

For applications in cells, cellular delivery of the precursor of the molecular sensor (or of the molecular sensor) of the present invention is aided/enabled in preferred embodiments by the cell penetrating properties of the calmodulin-binding peptides mediated by the presence of several positively charged amino acids.

For applications in neuroscience, trans blood-brain-barrier (BBB) uptake can be achieved via active transport (e.g. coupling to transferrin receptor or insulin receptor binding moieties such as antibodies) or after application of BBB opening techniques, such as ultrasound-mediated BBB opening, or pharmacological intervention, such as hyperosmolar shock.

The present invention provides the molecular sensor of the present invention *for use as a medicament.*

The present invention provides the molecular sensor of the present invention for use in a method of diagnosing and/or monitoring treatment of diseases causing changes in metal concentrations.

Thereby, the progress of a disease and/or the treatment of a disease can be monitored.

Preferably, a "disease causing changes in metal concentrations" is selected from:
(1) diseases in which calcium signaling is affected
   as in:
   neuropsychiatric diseases, such as epilepsy,
   stroke,
   brain damage,
   neurodegenerative diseases,
   states of altered neuronal processing, such as sleep, coma, anesthesia,
   intoxication,
   cardiovascular diseases, such as arrhythmias, cardiac ischemia and myocardial infarct,
   neuromuscular or muscular diseases,
(2) diseases in which calcium uptake, storage, utilization or excretion is affected as in
   endocrinological conditions, such as hyper/hypoparathyroidism,
   malnutrition and gastrointestinal diseases, such as malabsorption and diarrhea e.g. due to alcoholism,
   bone-related diseases, such as osteoporosis,
   kidney diseases and treatment with diuretics,
   osteoclastic processes of tumors or infections,
   diseases in which calcification in tissues occurs, such as cancers, including breast cancer,
   atherosclerotic alterations or inflammatory processes,
   due to other medical treatments.

Preferably, the imaging is real-time.

Preferably, the uses comprise imaging of tissues and bodily fluids, such as blood, urine, lymph or lymphatic drainage, cerebrospinal fluid, stool/feces, semen, saliva or mucous fluids, or a cell culture sample, such as derived from the human or non-human animal body, *ex vivo* tissue, cell culture.

*Methods for determining metal concentration and*/*or measuring metal concentration changes* The present invention provides *an in vitro* method for determining metal concentration and/or measuring metal concentration changes in a sample.

Said method comprises the steps of
(i) providing the precursor of a molecular sensor according to the present invention,
(ii) providing an active nucleus of said molecular sensor to the sample,
(iii) performing nuclear magnetic resonance (NMR) spectroscopy and imaging (MRS, MRI, Hyper-CEST) in a sample,
(iv) determining metal concentrations or metal concentration changes of or in the sample.

The active nucleus is preferably a spin-hyperpolarized nucleus, such as ¹²⁹Xe or other reversibly binding nuclei.

The provision of the active nucleus, such as hyperpolarized ¹²⁹Xenon, to the sample can be achieved via e.g. bubbling/dispersing it into solution.

Step (iii) can optionally comprise performing further measurement(s) to detect the further sensor moiety(moieties) (d).

Preferably, the sample is a biological fluid, such as blood, urine, lymph or lymphatic drainage, cerebrospinal fluid, stool/feces, semen, saliva or mucous fluids, or a cell culture sample, such as derived from the human or non-human animal body, *ex vivo* tissue, cell culture.

### Methods for diagnosing and/or monitoring treatment of diseases causing changes in metal concentrations

The present invention provides a method for diagnosing and/or monitoring treatment of diseases causing changes in metal concentrations.

### Said method comprises the steps of

(i) administering to the body of a patient or nun-human animal the precursor of a molecular sensor according to the present invention,
(ii) providing an active nucleus of said molecular sensor to the region or tissue of interest,
(iii) *performing in vivo* nuclear magnetic resonance (NMR) spectroscopy and imaging (MRS, MRI, Hyper-CEST),
   optionally performing further measurement(s) to detect the further sensor moiety(moieties),
(iv) determining metal concentrations or metal concentration changes of or in the body of said patient or nun-human animal.

Preferably, said method is in real-time.

In step (ii), the active nucleus is preferably a spin-hyperpolarized nucleus, such as ¹²⁹Xe or other reversibly binding nuclei.

Preferably, the active nucleus is hyperpolarized xenon gas, which can be e.g. inhaled.

The administration of the active nucleus, preferably the hyperpolarized ¹²⁹Xenon, can be achieved via administration through the breathing air (e.g. by inhalation), Xe mixture or injection of a Xe-saturated carrier solution such as saline solution, blood plasma etc.

Step (iii) can optionally comprise performing further measurement(s) to detect the further sensor moiety(moieties) (d).

As discussed above, a "disease causing changes in metal concentrations" is preferably selected from:
(1) diseases in which calcium signaling is affected
   as in:
   neuropsychiatric diseases, such as epilepsy,
   stroke,
   brain damage,
   neurodegenerative diseases,
   states of altered neuronal processing, such as sleep, coma, anesthesia,
   intoxication,
   cardiovascular diseases, such as arrhythmias, cardiac ischemia and myocardial infarct,
   neuromuscular or muscular diseases,
(2) diseases in which calcium uptake, storage, utilization or excretion is affected as in
   endocrinological conditions, such as hyper/hypoparathyroidism,
   malnutrition and gastrointestinal diseases, such as malabsorption and diarrhea e.g. due to alcoholism,
   bone-related diseases, such as osteoporosis,
   kidney diseases and treatment with diuretics,
   osteoclastic processes of tumors or infections,
   diseases in which calcification in tissues occurs, such as cancers, including breast cancer,
   atherosclerotic alterations or inflammatory processes,
   due to other medical treatments.

### Further description of preferred embodiments

The molecular sensor of the present invention differs from sensors described before, such as in Klippel *et al.,* (2014), Rose *et al.* (2014) or Witte *et al.* (2015), since they rely on increased uptake into the cells or specific binding to a cellular (surface) target in as much as the CEST effect for activating the image contrast is always available, even if the sensor is not yet bound to the target. In the molecular sensor of the invention the CEST effect is suppressed in the absence of the interaction with the target and gets unsuppressed in an analyte-dependent manner.

The present invention describes a sensor for Nuclear Magnetic Resonance (NMR) and Magnetic Resonance Imaging (MRI) based on spectral changes of the NMR signature (such as the NMR saturation transfer) of temporarily encapsulated hyperpolarized ¹²⁹Xenon caused by analyte-dependent changes of the local environment of a Xenon-binding molecule coupled interaction moieties, such as proteins or peptides. Such changes in the NMR signature can be a change in the resonance frequency (so-called chemical shift) or the intensity or width of a saturation response through chemical exchange saturation transfer onto free hyperpolarized Xe (Hyper-CEST). The Hyper-CEST effect is typically probed by radio frequencey (RF) saturation schemes, either continuous wave (cw) or pulsed irradiation.

The present invention relates to a novel analyte-triggered NMR signal turn on mechanism and specifically designed sensor compound that supports it.

The sensor of the present invention needs to provide the following functionalities:
(a) an active nucleus (e.g. xenon);
(b) a host for the active nucleus (e.g. xenon host (such as cryptophane, cucurbit[n]uril etc.),
(c) an NMR signal-modulating moiety (e.g. a peptide)
   that changes (e.g. decreases) the NMR saturation transfer signal of the active nucleus-host complex (e.g. suppresses saturation transfer from the otherwise accessible host by a specific conformation of the peptide in the host vicinity),
(d) an interaction moiety
   that can be tailored for specific interaction with an analyte or an environmental parameter of interest such that upon this interaction the NMR saturation transfer signal of the active nucleus-host complex is altered (under otherwise identical saturation transfer parameters such as RF power and duration),
(e) optionally, a contrast agent moiety or further sensor moiety
   that function as additional sensor(s) (e.g. another active nucleus-host complex such as cucurbit,) or optically detectable contrast agents, PET contrast agents, ultrasound contrast agents,) or actuators (such as photosensitizers, ultrasound contrast agents, magnetic structures).
(f) an optional solubilizing and/or biodistribution moiety
   that modifies the solubility and biodistribution of the active-nucleus host complex (e.g. cell permeability).

In a preferred embodiment of the invention, a xenon-binding cage-like compound called the "host" (such as cryptophane or similar entities, e.g. macrocyclic units like cucurbit[n]uriles, pillar[n]arenes, or self-assembling metal-organic cages etc.) is conjugated to a peptide (such as RS20, M13) that binds to an EF hand protein (such as calmodulin) in a calcium-dependent manner.

In the absence of the analyte (in this case calcium ions) the saturation transfer signal from the cryptophane-encapsulated hyperpolarized ¹²⁹Xenon is suppressed by the peptide acting as "the NMR-signal modulating moiety". If the analyte is added, the peptide binds tightly with the protein calmodulin and undergoes a conformational change, thereby turning on (unsuppressing) the saturation transfer signal in a calcium-dependent manner. The analyte-dependent NMR signal turn on of this preferred embodiment thus occurs as schematized in Figures 1 and 2.

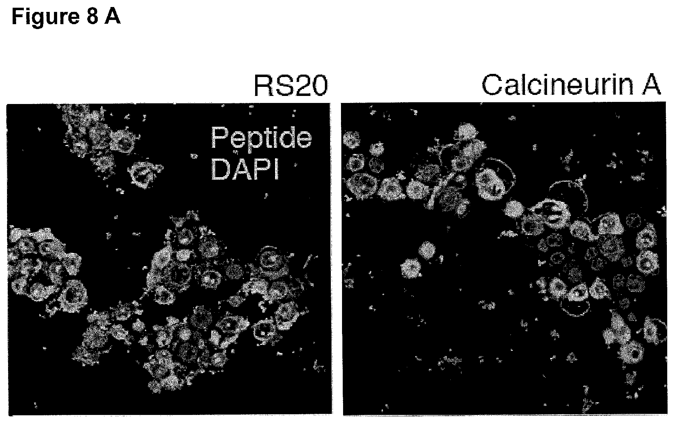

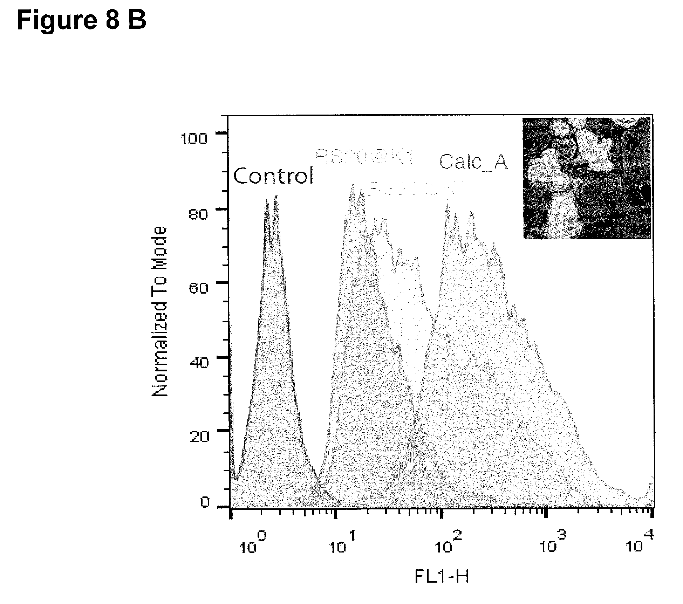

In the preferred embodiment, the peptide fulfills the function of NMR modulation and binding. It also enables cellular uptake (see Figure 8) thereby exerting function (f), as described above. Furthermore, the peptide also harbors a fluorophore that acts as secondary contrast agent or further sensor moiety (e) and may enhance the NMR signal suppression effect.

A particularly advantageous feature of a preferred embodiment of the sensor is a complete suppression of the CEST signal in the absence of calcium. As demonstrated in Figures 5 and 6, this previously unknown 'analyte-triggered turn on Hyper-CEST effect' depends on the specific choice of the peptide sequence and position of the cryptophane cage. Specifically, just shifting the cryptophane cage three amino acids more to the center of the RS20 peptide ("RS20-cage@K2") diminishes the CEST suppression and the amplitude of the signal change as compared with the sensor peptide ("RS20-cage@K1").

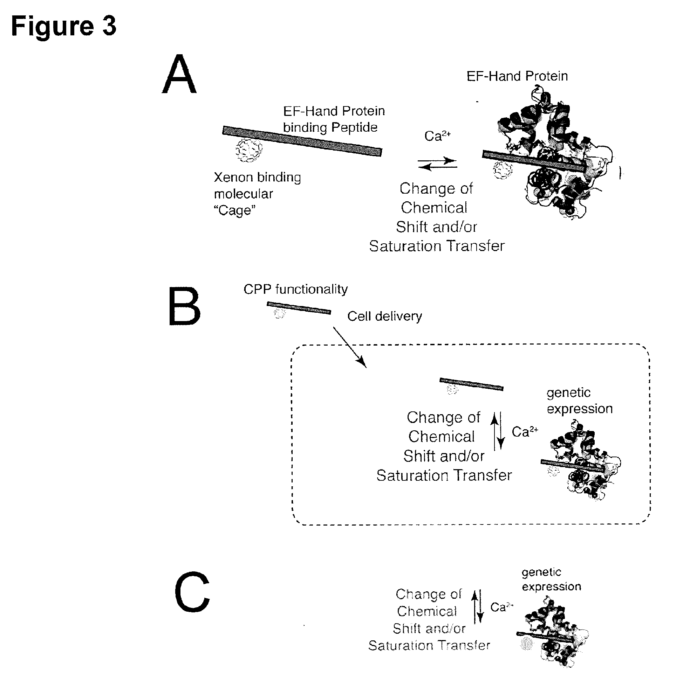

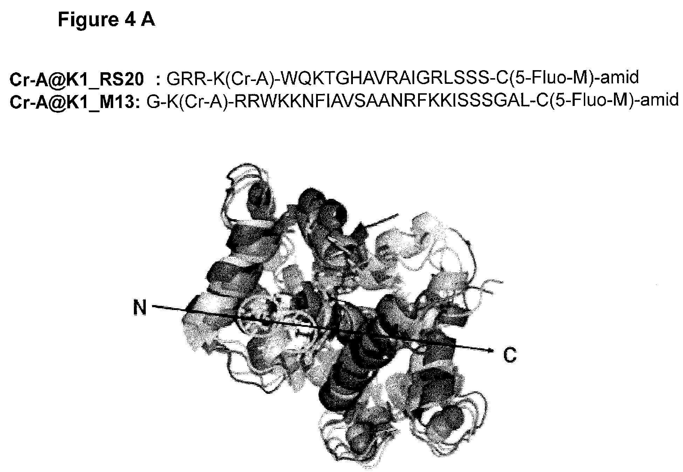

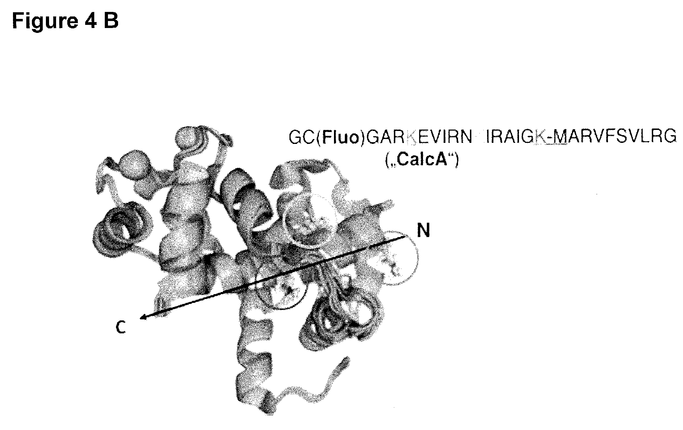

Aided by the well-characterized crystal structures of calmodulin with peptides (see Figures 3 and 4), we have thus identified the precise geometrical configuration of the sensor ensemble to enable a new turn-on hyper-CEST effect; a similar effect for any analyte has not been previously published or described.
A previously published Calcium sensor had a turn-on signal in the presence of Ca that was used to generate a ¹⁹F CEST response with specificity for Ca ions over Mg and Zn ions (Bar-Shir *et al.,* 2013). However, since this sensor did not employ spin-hyperpolarized Xe and therefore requires concentrations in the mM range. Moreover, it does not rely on a modulation of the the exchange kinetics of reversibly binding nuclei (Xe in our case) but rather on the kinetics of conformational changes of a chelate that comprises permanently bound ¹⁹F nuclei for NMR/MRI detection.
Whereas the cellular delivery of the hyper-CEST sensor described in this application can occur over hours to be later 'interrogated' by rapdily administered hyperpolarized Xenon, sensors that work via a chemical shift of a hyperpolarized ¹³C or ¹⁵N moiety only provide increased signal for very short time interval limited by the relaxation rates of the heteronuclei (Nonaka *et al.,* 2015; Hata *et al.,* 2013). Synthetic compounds have been made to sense toxic metals but via a conventional chemical shift mechanism (Tassali *et al.,* 2014) not the CEST un(suppression) described herein here.

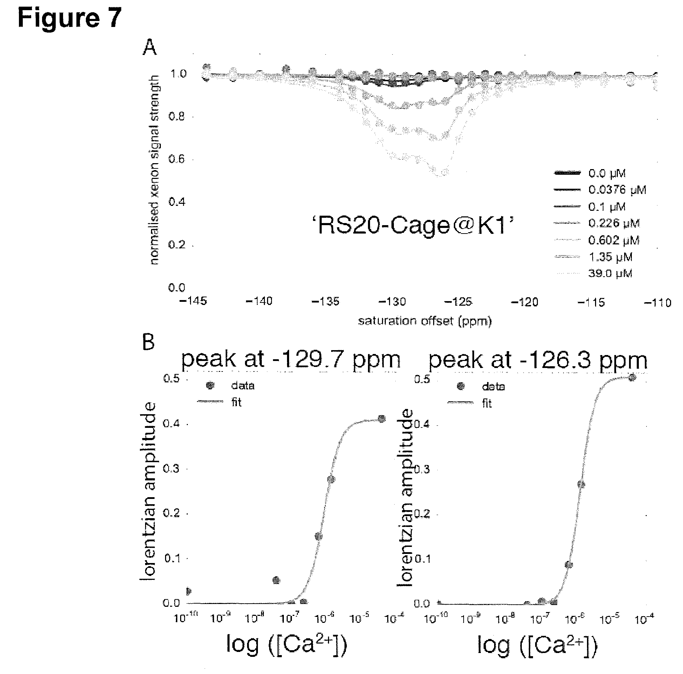

Using this robust turn-on CEST effect, we can demonstrate quantitative calcium detection in the lower micromolar range with an excellent dynamic range for physiological concentrations during signal transduction processes (Figure 7).

We furthermore conducted confocal microscopy studies confirming that the fluorophore-labeled peptides are readily taking up into cells (Figure 8A and B).

The means and methods of the present invention offer the following unique combination of advantages:
(1) A high signal-to-noise ratio (SNR) is provided due to the CEST-suppression in the absence of the analyte and the strong analyte-triggered NMR Signal turn-on obtained from hyperpolarized Xenon with Chemical Exchange Saturation Transfer (Hyper-CEST);
   and
(2) Fast delivery of the Xe-carrying unit (the host) is not necessary, unlike in other hyperpolarization methods based on *e.g.* compounds containing ¹³C or ¹⁵N with short decay rates.

Instead, the delivery of the xenon-binding host to the target organ (including the brain) and into the cell can occur at slow kinetics. The interrogation of the distribution and analyte-dependent state of the host can then take place in a second step within the optimal time windows defined by the decay rates of the hyperpolarized signal. The administration of the hyperpolarized ¹²⁹Xenon can be achieved for *in vitro* applications via bubbling/dispersing it into solution or via administration through the breathing air/Xe mixture or injection of a Xe-saturated carrier solution such as saline solution, blood plasma etc. for *preclinical in vivo* studies.

The means and methods described herein could be used for *in vitro* detection of calcium in opaque specimens.

For applications in cells, cellular delivery of the scaffold-host conjugate (peptide-cage) is enabled by the cell penetrating properties of the calmodulin-binding peptides mediated by the presence of several positively charged amino acids. For applications in neuroscience, trans blood-brain-barrier (BBB) uptake can be achieved via active transport (e.g. coupling to transferrin receptor or insulin receptor binding moieties such as antibodies) or after application of BBB opening techniques, such as ultrasound-mediated BBB opening, or pharmacological intervention, such as hyperosmolar shock.

Specificity for genetically defined cellular populations can be achieved by genetically expressing a variant of the calcium-binding protein, such as calmodulin, that exhibits a specific affinity for the peptide-cage construct being delivered to the cell (Figure 3B). This can be achieved by e.g. using high-affinity interacting moieties, such as SpyTag/SpyCatcher, Biotin/Avidin or antiparallel homodimeric protein interfaces. Alternatively, the xenon-binding host molecule can be delivered across the cellular membrane to find the specific genetically expressed calcium-binding protein such as a calmodulin-peptide fusion.

The sensitivity of the calcium sensor can be tuned by using variants of the calcium protein such as calmodulin with higher or lower affinities for calcium. Also orthogonal calmodulin/peptide pairs could be used alternatively to ensure orthogonality, i.e. that do only interact with each other but do not have high affinities for the respective endogenous binding partners.
Alternatively, the sensitivity of the sensor can be tuned by using different proteins that exhibit lower affinity, such as proteins interacting with calcium at higher concentrations present in *e.g.* synaptic vesicles (*e.g.* C2A domain containing, lipid-binding proteins). This allows for tuning the sensor to extracellular calcium concentrations, thus avoiding the need for cellular delivery of the agent.

Similarly, the means and methods described herein can be extended to sensing other external stimuli, such as the presence of other metals, and changes in environmental parameters, such as pH, by using the respective alternative protein-peptide pairs.

A ratiometric readout can be achieved by addition of a second Xe host (e.g. cucurbit or cryptophane) whose environment does not change as a function of calcium binding (or other of the above-mentioned stimuli) such that it can be used to normalize for the concentration of the sensor.

Furthermore, multimodal calcium sensing can be achieved by attaching an environmentally sensitive fluorophore to the calmodulin-binding peptide. Similarly, localization of the sensor by an independent read-out can be built in via a fluorophore, an MRI agent such as a T₁ agent represented by gadolinium DPTA (or similar chelates of paramagnetic ions), or a radioactive probe for PET detection. This multimodal readout is made possible by the precise knowledge of the three-dimensional structure of a primary analyte such as calmodulin that affords the extension of the peptidic scaffold out of the binding pocket; external modifications by the modifiers mentioned above can thus be realized without interfering with the calcium-mediated peptide binding mechanisms.

As stated above, the moiety attached in addition to the xenon-binding cage (such as a chelated transition metal or a molecular such as a planar fluorophore) can influence the chemical shift of xenon thereby supporting its calcium-dependent change via peptide-protein binding (that e.g. increases the average distance between the cryptophane cage and the additional moiety)

The semi-genetic method featuring a genetically expressed protein and synthetically modified peptide to be delivered to the sample (either extracellularly or intracellularly) could also be made a fully-genetic solution by genetically expressing a Xenon-host (e.g. gas binding proteins or self-assembling macromolecular structures) with function similar to that of the synthetic cryptophane cage.

The calcium sensor of the present invention has a few orders of magnitude higher sensitivity in terms of the signal to noise ratio over any existing calcium sensor for NMR/MRI (see e.g. Schröder *et al.,* 2006; Schröder *et al.,* 2008-1; Schröder *et al.,* 2008-2; Kunth *et al.,* 2012).

This is caused by the 'analyte-triggered CEST effect', as described herein. The sensor has furthermore an optimal dynamic range to detect relevant intracellular concentrations of calcium and we show that it easily enters into cells in stark contrast to many of the other concepts for NMR sensors (see e.g. Caravan 2006; Caravan *et al.,* 1999).

The calcium sensor of the present invention utilizes Xenon hyperpolarization and the hyper-CEST mechanism that has been described before by Schröder et al. (2006).

The very specific advantageous properties of the means and methods of the invention are:
- high sensitivity,
- high specificity,
- cell penetrance,
- high SNR through analyte-triggered CEST effect,
that will enable *in vitro, ex vivo* and *in vivo* use of the analyte sensor (calcium sensor) in biomedical applications.

The molecular calcium sensor specified in the present invention disclosure has a broad range of possible applications in *in vitro, in vivo* and *ex vivo* detection of calcium concentration changes as well as measurements of spatiotemporal distribution of calcium via Nuclear Magnetic Resonance (NMR) spectroscopy and imaging (MRS, MRI, Hyper-CEST).

These photon-independent detection methods have great advantages over methods that rely on fluorescence, absorbance or bioluminescence for measuring metal concentration changes in opaque or turbid samples and live organ(ism)s. As compared with existing NMR-based metal-sensitive contrast agents, the present invention disclosure describes the generation of a molecular cage-based sensor that can be interrogated via addition of hyperpolarized xenon generating orders of magnitude higher NMR signal. This increased SNR is a necessary precondition to perform *in vivo* measurements of spatiotemporal metal distributions, preferably with direct quantification via ratiometric capabilities and preferably with the ability identify specific metals via their characteristic chemical shift.

The preferred field of application for the metal-biosensors described herein is *in vivo* imaging of calcium distributions in preclinical animal models and humans, in particular for diagnosing and/or monitoring treatment of diseases associated with changes in metal concentrations, such as diseases:
in which calcium signaling is affected
   as in neuropsychiatric diseases such as epilepsy, stroke, brain damage, neurodegenerative diseases, states of altered neuronal processing such as sleep, coma, anesthesia, intoxication,
   cardiovascular diseases, such as arrhythmias, cardiac ischemia and infarct, neuromuscular or muscular diseases,
in which calcium uptake, storage, utilization or excretion is affected
   as in endocrinological conditions such as hyper/hypoparathyroidism, malnutrition and gastrointestinal diseases such as malabsorption and diarrhea e.g. due to alcoholism, bone-related diseases such as osteoporosis, kidney diseases and treatment with diuretics, osteoclastic processes of tumors or infections, diseases in which calcification in tissues occurs such as cancers including breast cancer, atherosclerotic alterations or inflammatory processes, due to other medical treatments

The molecular sensor can furthermore be applied in, *ex vivo* (pre)clinical imaging of tissues and bodily fluids (such as blood, urine, feces, CSF, lymphatic drainage) and explanted tissues or cells, and *in in vitro* measurement and imaging in biomedical (cell and tissue cultures) or environmental samples (such as metal screening in e.g. water, air, soil, plants, food).

The following examples and drawings illustrate the present invention without, however, limiting the same thereto.

### BRIEF DESCRIPTION OF THE DRAWINGS

**Figure 1****:** *Schematic representation of on Turn-on Mechanism.*
   Visualization of the different moieties of the sensor systems that enable the analyte-dependent activation of the NMR signal that is suppressed in the absence of the target condition e.g. presence of the analyte and unsuppressed if this condition is fulfilled.
**Figure 2****:** *Calcium-dependent spectral changes of temporally encapsulated hyperpolarized 129Xenon.*
   (A) The presence of a Xe host-bearing peptidic sequence (labelled with an optical reporter for fluorescence co-registration) is revealed by a certain NMR saturation transfer response along the chemical shift dimension when observing free xenon after RF saturation of bound Xe. The intensity of the spectral response can be weak or strong and does not change significantly upon addition of calmodulin. Only further presence of calcium as analyte induces a conformational change of the peptidic scaffold around the captured Xe host that induces spectral changes for saturation of temporarily bound xenon. The peptidic scaffold therefore serves as the NMR modulating moiety, CaM interacts with the interaction moiety in a calcium-dependent manner. These are identified as altered intensity, frequency, or width of the saturation response or any combinations thereof (B).
**Figure 3****:** *Scheme depicting the in vitro and in vivo applications of the calcium sensor.*
   Dependent on the calcium concentration, a specific peptidic scaffold will be tightly bound by an EF-hand protein such as calmodulin. Hyperpolarized Xenon can temporarily bind to a molecular (cage-shaped) host conjugated to the peptidic scaffold that suppresses the NRM signal in the absence of the analyte calcium. In the presence of calcium, the peptide will bind to calmodulin and Xenon will thus sample a different molecular environment in a calcium-dependent manner resulting in a different NMR signature expressed by the chemical shift and/or saturation transfer (Hyper-CEST).
   (A) This "analyte-dependent Hyper-CEST turn-on effect" can thus be used *for in vitro* or *ex vivo* detection of calcium by NMR/MRI. (B) For cellular *and in vivo* calcium detection, an engineered variant of the protein can be genetically expressed that binds to the cage-modified peptide delivered to the cell again resulting in the NMR signature.
   Alternatively, the binding partner for the primary analyte can be a surface-expressed protein or lipid modification (not drawn). (C) The entire sensor including the Xenon host is genetically expressed.
**Figure 4****:** *Calcium-dependent docking of peptides to the calcium-binding protein calmodulin.*
   As an example for calcium-dependent protein-peptide binding, the 3D structure of the EF hand protein calmodulin is shown bound to the peptide RS20 that has high similarity to M13 (the orientation of the peptide is indicated by the arrow). The lysine residues used for attachment of the Xenon-binding cage-like molecule cryptophane are underlined (color corresponding to the highlighted residues in the scheme). The position for attaching a fluorophore, a chelated transition metal or PET agent is indicated by the letters ,Fluo' **(A).** The alternative peptide calcineurin A is depicted that has opposite directionality. The cage-conjugated calcineurin does not exhibit a change in the CEST effect upon binding to calmodulin. Same color coding and highlighting of residues as above **(B).**
**Figure 5****:** *CEST signal for different cryptophane-conjugated peptides in the absence and presence of calcium.*
   Hyperpolarized Xenon was bubbled into buffered (MOPS) solutions containing different cryptophane bearing peptides indicated in the respective subfigures (1 µM) and calmodulin (3 µM) in the absence and presence of 100 µM calcium and CEST spectra were obtained on a 9.4 T Bruker AV 400 wide bore NMR spectrometer. (E) Effects of C-terminal portion of RS20 on the NMR-signal modulation. Replacement of the maleimide-Fluorescein by a maleimide capping group (NEM) partially unsuppresses the CEST effect. (These data identify in particular the C-terminus of RS20 and M13 to be functioning as the NMR-modulating moiety, whereas the N-terminus is mainly engaged in interacting (interaction moiety) with CaM).
**Figure 6****:** *Conformational Changes of NMR-modulating moiety of the Sensor System* (A) Circular Dichroism data showing how with 20% TFE, an α-helical structure is promoted, but not fully induced. At 50% TFE, the induction an α-helical structure is enforced. (B) Corresponding normalized CEST spectra showing that the CEST effect is strongly switched on when the peptide is forced to assume α-helical structure.
**Figure 7****:** *Quantitative Detection of Calcium through turn-on effect.*
   CEST spectra (A) and binding curves (B) of the sensor peptide RS20-cage@K1 (2 µM) in the presence of 10 µM calmodulin are shown for calcium concentrations ranging from 0 to 39 µM (MOPS buffer).
**Figure 8****:** *Cellular uptake of cage-conjugated peptides.*
   (A) Cage-conjugated peptides that were also labeled with fluorescein (25 µM) were incubated on HEK293 cells for 20 minutes followed by confocal microscopy imaging (nuclear counterstain with DAPI). **(B)** Analysis of FACS experiments with cells incubated with different peptides modified with cryptophane cages and fluorescein indicating labeling of the cells with the fluorescent peptides (sequences as indicated in Figure 2; @K# indicates the lysine residue number used for conjugation of the cryptophane cage). The inset shows a cell after uptake of the fluorescent RS20@K1 peptide (nuclear counterstain with Hoechst dye). The cellular uptake of similar EF-protein-binding peptides is likely due to their high scores on prediction tools for cell-penetration such as CPPpred or CellPPD.

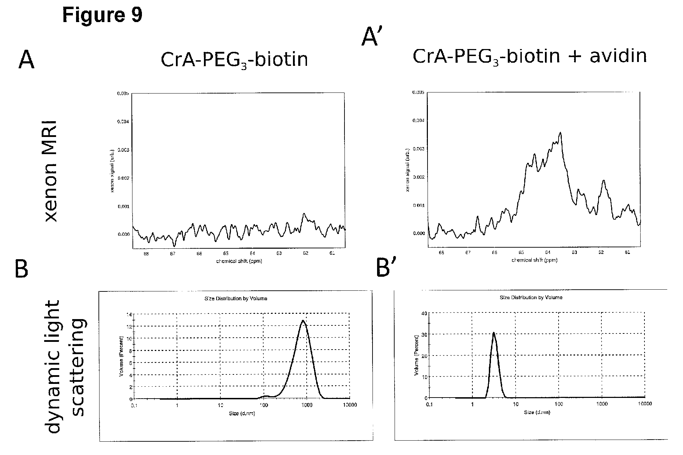

**Figure 9****:** *Turn-on mechanism from biotinylated-PEG-cryptophane cage.*
   The figure displays a switch in the CEST signal from hyperpolarized Xenon interacting with a biotinylated cryptophane cage (CrA-PEG3-biotin) depending on the absence **(A)** or presence **(B)** of the high affinity binding partner avidin. The lower row shows the corresponding Dynamic Light Scatter (DLS, averaged data from 850 nm) results showing a reduction of size of the CrA-PEG3-biotin upon binding to avidin most likely as a result of a change in the agglomeration state mediated by a solubility switch.

### EXAMPLES

### EXAMPLE 1

### Materials and Methods

We have generated peptides of the specific sequences as listed above and have attached a cryptophane cage at exactly the positions indicated to obtain a reversible CEST suppression effect under baseline conditions (in the absence of the analyte) as shown in the figures.

As can be seen in Figure 5, a specific amino acid composition is essential for reversible CEST suppression and unsuppression of the Ca²⁺-sensor. Attachment of the cryptophane cage at the first lysine of the peptide RS20 exhibits a complete CEST suppression and a strong, Ca²⁺-dependent CEST unsuppression (Figure 5 A). Similar effects can be observed for the peptide M13 when increasing amounts of Ca²⁺ (0-100 µM) are added (Figure 5 D). However, the CEST suppression and Ca²⁺-mediated unsuppression is weaker if the cryptophane cage is attached at the third lysine (@K3, Figure 5B). Moreover, a specific calcineurin A peptide with an attachment of the cryptophane cage at a central position ARKEVIRNKIRAIGK-CrytophaneCage-MARVFSVLR (SEQ ID NO. 3) does neither show a suppression nor an unsuppression. (Figure 5C).
In addition, slight modifications of the C-terminus (e.g. replacement of the fluorescein by a capping group (N-ethylmaleimide) diminishes the CEST suppression effect (Figure 5E, blue curve as compared to the black curve in 5D).

To generate the data shown in the Figures, the following general procedures were applied.

The production, delivery and application of hyperpolarized Xe for NMR and MRI studies has been described previously including a full list of materials (Witte *et al.,* 2012; Witte *et al.,* 2014). Typically, hyperpolarized ¹²⁹Xe (20 % polarization and more) is generated by spin exchange optical pumping using a 150 W continuous wave laser (795 nm, 0.5 nm bandwidth) in a custom-designed continuous flow setup at 4.5 bar absolute pressure using a gas mixture of 5 % Xe (26.4% natural abundance of ¹²⁹Xe), 10% N₂, and 85% He.

Experiments benefit from high field MRI scanners because of the increased spectral resolution for resolving the CEST response. As an example, a 9.4 T NMR spectrometer can be used for the NMR experiments. It requires gradient coils for MR imaging and a variable temperature unit for adjusting the sample temperature. A 10 mm inner-diameter double resonant probe (¹²⁹Xe (110 MHz) and ¹H (400 MHz)), can be used for excitation and detection.
The freshly hyperpolarized xenon gas mix is directly bubbled into solution for ca. 15 seconds at a total flow rate of 0.1 SLM followed by a 1 s delay (to allow possible remaining bubbles to collapse) before signal acquisition. To reduce foaming, 0.1 % of Pluronic^{®} L81 may be added to the sample. After bubbling, a saturation RF pulse is irradiated at the desired frequency and the signal of free Xe in solution is observed. ¹²⁹Xe Hyper-CEST MR images can be acquired using averages of a RARE sequence with a slice-selective 90° gaussian shaped excitation pulse and high RARE factor (up to 32 for a 32x 32 matrix size) due to sufficiently slow T2 relaxation. Two images are acquired, one with an off-resonant saturation pulse for control and one with an on-resonant saturation pulse to induce signal loss in the vicinity of the sensor. Hyper-CEST MR images are obtained by a pixel-wise subtraction of the noise corrected off-and on-resonant images. All data analysis was performed with MATLAB®.

### EXAMPLE 2

As an additional preferred instantiation of the CEST unsuppression effect, Figure 9 shows results from a Crytpophane cage modified with a short polyethylene glycol (PEG) chain that contains a biotin moiety at the end. Binding of this compound to avidin (Figure 9A') leads to a CEST unsuppression that is most likely due to a solubility switch as indicated by the large apparent size of the compound (as measured by Dynamic Light Scattering) in the absence of avidin (most likely indicating agglomerates) that gets reduced upon binding to avidin.

The features disclosed in the foregoing description, in the claims and/or in the accompanying drawings may, both separately and in any combination thereof, be material for realizing the invention in diverse forms thereof.

### REFERENCES

Afshar M, Caves LS, Guimard L, Hubbard RE, Calas B, Grassy G and Haiech J (1994) Investigating the high affinity and low sequence specificity of calmodulin binding to its targets. _J Mol Biol_244:554-571.
Aldiri T, Marciano D, Cohen Y. Potential 129Xe-NMR biosensors based on secondary and tertiary complexes of a water-soluble pillar[5]arene derivative. Chem. Commun., 2013,49, 7082-7084. doi: 10.1039/C3CC43253J.
Barnes JA and Gomes AV (1995) PEST sequences in calmodulin-binding proteins. _Mol Cell Biochem_149-150:17-27.
Bar-Shir A, Gilad AA, Chan KW, Liu G, van Zijl PC, Bulte JW, McMahon MT. Metal ion sensing using ion chemical exchange saturation transfer 19F magnetic resonance imaging. J Am Chem Soc. 2013;135(33):12164-7. doi: 10.1021/ja403542g. Epub 2013 Aug 6.
Caravan P, Ellison JJ, McMurry TJ, Lauffer RB. Gadolinium(III) Chelates as MRI Contrast Agents: Structure, Dynamics, and Applications. Chem Rev. 1999 Sep 8;99(9):2293-352. Caravan P. Strategies for increasing the sensitivity of gadolinium based MRI contrast agents. Chem Soc Rev. 2006;35(6):512-23.
Carter, K. P., Young, A. M., & Palmer, A. E. (2014). Fluorescent Sensors for Measuring Metal Ions in Living Systems. Chemical Reviews, 114(8), 4564-4601. doi:10.1021/cr400546e
Durham, A. C. H., & Walton, J. M. (1983). A survey of the available colorimetric indicators for Ca2+ and Mg2+ ions in biological experiments. Cell Calcium, 4(1), 47-55. doi:10.1016/0143-4160(83)90048-9.
Erickson-Viitanen S and DeGrado WF (1987) Recognition and characterization of calmodulin-binding sequences in peptides and proteins. _Methods Enzymol_139:455-478*.*
Hata R, Nonaka H, Takakusagi Y, Ichikawa K, Sando S. Design of a hyperpolarized (15)N NMR probe that induces a large chemical-shift change upon binding of calcium ions. Chem Commun (Camb). 2015;51(61):12290-2. doi: 10.1039/c5cc04597e.
Klippel S, Döpfert J, Jayapaul J, Kunth M, Rossella F, Schnurr M, Witte C, Freund C, Schröder L. Cell tracking with caged xenon: using cryptophanes as MRI reporters upon cellular internalization. Angew Chem Int Ed Engl. 2014; 53(2):493-6. doi: 10.1002/anie.201307290. Epub 2013 Dec 4.
Kunth M, Döpfert J, Witte C, Rossella F, Schröder L. Optimized use of reversible binding for fast and selective NMR localization of caged xenon. Angew Chem Int Ed Engl. 2012;51(33):8217-20. doi: 10.1002/anie.201202481.
Kunth M, Witte C, Schröder L.Continuous-wave saturation considerations for efficient xenon depolarization. NMR Biomed. 2015;28(6):601-6. doi: 10.1002/nbm.3307. Epub 2015 Apr 21.
Looger, L. L. (2013). Genetically encoded calcium indicators for multi-color neural activity imaging and combination with optogenetics, 1-29. doi:10.3389/fnmol.2013.00002/abstract
Lowery TJ, Rubin SM, Ruiz EJ, Pines A, Wemmer DE. Design of a conformation-sensitive xenon-binding cavity in the ribose-binding protein. Angew Chem Int Ed Engl. 2004;43(46):6320-2.
Nonaka H, Hata R, Doura T, Nishihara T, Kumagai K, Akakabe M, Tsuda M, Ichikawa K, Sando S.A platform for designing hyperpolarized magnetic resonance chemical probes. Nat Commun. 2013;4:2411. doi: 10.1038/ncomms3411.
Ntziachristos, V. (2010). Going deeper than microscopy: the optical imaging frontier in biology. Nature Methods, 7(8), 603-614. doi:10.1038/nmeth.1483
Rose HM, Witte C, Rossella F, Klippel S, Freund C, Schröder L. Development of an antibody-based, modular biosensor for 129Xe NMR molecular imaging of cells at nanomolar concentrations. Proc Natl Acad Sci U S A. 2014; 111(32):11697-702. doi: 10.1073/pnas.1406797111. Epub 2014 Jul 28.
Rubin SM, Lee SY, Ruiz EJ, Pines A, Wemmer DE. Detection and characterization of xenon-binding sites in proteins by 129Xe NMR spectroscopy. J Mol Biol. 2002;322(2):425-40.
Schröder L, Lowery TJ, Hilty C, Wemmer DE, Pines A. Molecular imaging using a targeted magnetic resonance hyperpolarized biosensor.Science. 2006;314(5798):446-9.
Schröder L. (2007) Xenon im Käfig für die Krebsdiagnose. Spektrum d Wissenschaft. April 2007.
Schröder L, Meldrum T, Smith M, Lowery TJ, Wemmer DE, Pines A. Temperature response of 129Xe depolarization transfer and its application for ultrasensitive NMR detection. Phys Rev Lett. 2008;100(25):257603. (2008-1)
Schröder L, Chavez L, Meldrum T, Smith M, Lowery TJ, Wemmer DE, Pines A. Temperature-controlled molecular depolarization gates in nuclear magnetic resonance. Angew Chem Int Ed Engl. 2008;47(23):4316-20. doi: 10.1002/anie.200800382. (2008-2)
Tassali, N., Kotera, N., Boutin, C., Léonce, E., Boulard, Y., Rousseau, B., Dubost, E., Taran, F., Brotin, T., Dutasta, J.-P., Berthault, P., 2014. Smart detection of toxic metal ions, Pb2+ and Cd2+, using a 129Xe NMR-based sensor. Anal. Chem. 86, 1783-1788.
Vetter SW and Leclerc E (2003) Novel aspects of calmodulin target recognition and activation. _Eur J Biochem_270:404-414*.*
Viale, A. et al. Hyperpolarized agents for advanced MRI investigations (2009). Q. J. Nucl. Med. Mol. Imaging 53, 604-617.
Viale, A. & Aime, S. (2010). Current concepts on hyperpolarized molecules in MRI. Current Opinion in Chemical Biology 14, 90-96.
Witte C, Kunth M, Döpfert J, Rossella F, Schröder L. Hyperpolarized xenon for NMR and MRI applications. J Vis Exp. 2012;(67). pii: 4268. doi: 10.3791/4268.
Witte C, Kunth M, Rossella F, Schröder L. Observing and preventing rubidium runaway in a direct-infusion xenon-spin hyperpolarizer optimized for high-resolution hyper-CEST (chemical exchange saturation transfer using hyperpolarized nuclei) NMR. J Chem Phys. 2014;140(8):084203. doi: 10.1063/1.4865944.
Witte C., Martos V, Rose HM, Reinke S, Klippel S, Schröder S and Hackenberger CPR. Xenon-MRT an lebenden Zellen mit Hyper-CEST-Biosensoren fur metabolisch markierte Glykane an der Zelloberfläche. Angew. Chem. 2015; 127(9): 2848-2852.

## Claims

1. A precursor for a molecular sensor for determining analyte concentrations and/or measuring analyte concentration changes
comprising
(a) a host for an active nucleus,
preferably a cage-like molecule,
wherein said host enables at least a transient binding of said active-nucleus that produces a detectable NMR signal when the sensor binds to the analyte,
(b) an NMR-modulating moiety,
wherein said NMR-modulating moiety changes the resonance frequency or the chemical exchange saturation transfer (CEST) signal of the active nucleus-host complex,
and
(c) an interaction moiety,
wherein said interaction moiety specifically responds to an environment parameter (such as temperature or viscosity), an analyte or a target molecule that binds the analyte
or said interaction moiety specifically binds to a target molecule in an analyte-dependent manner,
wherein the analyte is preferably a metal, more preferably calcium, or a protein.

2. A molecular sensor for determining analyte concentrations and/or measuring analyte concentration changes in a cell,
comprising
an active nucleus,
preferably xenon, more preferably hyperpolarized xenon,
and the precursor of claim 1,
wherein the analyte is preferably a metal, more preferably calcium, or a protein.

3. The molecular sensor of claim 2, which is assembled inside a cell or tissue,
wherein the active nucleus is delivered across the cellular membrane and the host is delivered across the cellular membrane or is expressed by said cell,
wherein said NMR-modulating moiety is preferably expressed by said cell,
and wherein said interaction moiety is preferably expressed by said cell.

4. The precursor or the molecular sensor of any one of claims 1 to 3, wherein said host is a xenon host, preferably selected from a cryptophane, cucurbit[n]uriles, pillar[n]arenes, or self-assembling metal-organic cages,
including genetically encoded compartments consisting of proteins, peptides, DNA and RNA.

5. The precursor or the molecular sensor of any one of claims 1 to 4, wherein the NMR-modulating moiety (b) and the interaction moiety (c) are attached to each other or form a joint moiety,
preferably the NMR-modulating moiety (b) and the interaction moiety (c) are comprised in or consist of a protein or peptide, or a linker,
more preferably comprising or consisting of a peptide selected from RS20, calcineurin A, M13, or another CaM-binding peptide, or a linker , such as polyethylene glycol (PEG).

6. The precursor or the molecular sensor of any one of claims 1 to 5, wherein the NMR-modulating moiety (b) and the interaction moiety (c) comprises or is the peptide RS20 or the pepide M13 that specifically binds to an EF hand protein, such as calmodulin, in a calcium-dependent manner,
preferably comprising the amino acid sequence selected from SEQ ID NOs. 1 to 3.

7. The precursor or the molecular sensor of any one of claims 1 to 6, wherein the NMR-modulating moiety (b) and the interaction moiety (c), which are preferably attached to each other, more preferably comprise or consist of a peptide as defined in claim 5 or 6, decrease the CEST signal of the active nucleus,
wherein, preferably, the CEST signal from the otherwise accessible host is (reversibly) suppressed by a specific conformation of the NMR-modulating moiety (b) in the host vicinity, and the interaction of the interaction moiety (c) with a target molecule in an analyte-dependent manner unsuppresses the CEST signal.

8. The precursor or the molecular sensor of any one of claims 1 to 7, wherein the host (a), the NMR-modulating moiety (b) and the interaction moiety (c) are attached to each other, such as
via an amino acid side chain of the peptide, e.g. the side chain of a lysine,
via a linker or tether.

9. The precursor or the molecular sensor of any one of claims 1 to 8, further comprising
(d) a further sensor moiety,
- a further active nucleus-host complex,
- contrast agent(s),
such as optically detectable contrast agents, PET contrast agents, ultrasound contrast agents),
- chromophore(s) and/or fluorophore(s),
such as environmentally sensitive fluorophores,
e.g. temperature, viscosity, pH, redox, metal-sensitive fluorophore- or chromophore-based sensors sensitive to electromagnetic or mechanical waves, particle, irradiation,
- MRI or PET agent(s) or chelated transition metal(s),
such as gadolinium DPTA, macrocycles,
- actuator(s),
such as photosensitizers, magnetic structures,
- nanostructures,
such as a gas-filled microbubble, a paramagnetic nanoparticle or gold particle,
- absorber
- or combinations thereof;
and/or
(e) a solubilizing and/or biodistribution moiety;
and/or
(f) further interacting moiety or moieties.

10. Use of a molecular sensor of any one of claims 2 to 9 for
- determining analyte concentrations and/or measuring analyte concentration changes,
- *in vitro, ex vivo* and *in vivo* measurements of spatiotemporal analyte distributions,
- *in vivo* imaging of analyte distributions in preclinical animal models and humans, in particular for diagnosing and/or monitoring treatment of diseases associated with changes in metal concentrations,
wherein the analyte is preferably a metal, more preferably calcium,
and/or comprising nuclear magnetic resonance (NMR) spectroscopy and imaging (MRS, MRI, Hyper-CEST),
optionally comprising multimodal detection of further sensor moiety/moieties, preferably as defined in claim 9, via absorbance/transmission, reflection, fluorescence or optoacoustic or ultrasound measurements and imaging.

11. The use according to claim 10, comprising
- *- ex vivo* (pre)clinical imaging of tissues and bodily fluids,
such as blood, urine, lymph or lymphatic drainage, cerebrospinal fluid, stool/feces, semen, saliva or mucous fluids, or a cell culture sample, such as derived from the human or non-human animal body, *ex vivo* tissue, cell culture,
- *in vitro* measurement and imaging in biomedical or environmental samples, such as in cell and tissue cultures, metal screening in e.g. water, air, soil, plants, food,
and/or comprising determining further analyte(s).

12. The molecular sensor of any one of claims 2 to 9 for use as a medicament.

13. The molecular sensor of any one of claims 2 to 9 for use in a method of diagnosing and/or monitoring treatment of diseases causing changes in metal concentrations,
wherein a disease causing changes in metal concentrations is preferably selected from:
- diseases in which calcium signaling is affected
as in:
neuropsychiatric diseases, such as epilepsy, stroke,
brain damage,
neurodegenerative diseases,
states of altered neuronal processing, such as sleep, coma, anesthesia, intoxication,
cardiovascular diseases, such as arrhythmias, cardiac ischemia and myocardial infarct,
neuromuscular or muscular diseases,
- diseases in which calcium uptake, storage, utilization or excretion is affected
as in
endocrinological conditions, such as hyper/hypoparathyroidism,
malnutrition and gastrointestinal diseases, such as malabsorption and diarrhea e.g. due to alcoholism,
bone-related diseases, such as osteoporosis,
kidney diseases and treatment with diuretics,
osteoclastic processes of tumors or infections,
diseases in which calcification in tissues occurs, such as cancers, including breast cancer,
atherosclerotic alterations or inflammatory processes,
due to other medical treatments,
preferably comprising imaging of tissues and bodily fluids,
such as blood, urine, lymph or lymphatic drainage, cerebrospinal fluid, stool/feces, semen, saliva or mucous fluids, or a cell culture sample, such as derived from the human or non-human animal body, *ex vivo* tissue, cell culture.

14. *An in vitro* method for determining metal concentration and/or measuring metal concentration changes in a sample, comprising the steps of
(i) providing the precursor of a molecular sensor according to any one of claims 1 to 9,
(ii) providing an active nucleus of said molecular sensor to the sample,
preferably a spin-hyperpolarized nucleus,
(iii) performing nuclear magnetic resonance (NMR) spectroscopy and imaging (MRS, MRI, Hyper-CEST) in a sample,
optionally performing further measurement(s) to detect the further sensor moiety(moieties) (e),
(iv) determining metal concentrations or metal concentration changes of or in the sample,
wherein the sample is preferably a biological fluid, such as blood, urine, lymph or lymphatic drainage, cerebrospinal fluid, stool/feces, semen, saliva or mucous fluids, or a cell culture sample, such as derived from the human or non-human animal body, *ex vivo* tissue, cell culture.

15. A method for diagnosing and/or monitoring treatment of diseases causing changes in metal concentrations, preferably in real-time, comprising the steps of
(i) administering to the body of a patient or nun-human animal the precursor of a molecular sensor according to any one of claims 1 to 9,
(ii) providing an active nucleus of said molecular sensor to the region or tissue of interest,
preferably a spin-hyperpolarized nucleus,
(iii) *performing in vivo* nuclear magnetic resonance (NMR) spectroscopy and imaging (MRS, MRI, Hyper-CEST),
optionally performing further measurement(s) to detect the further sensor moiety(moieties) (e),
(iv) determining metal concentrations or metal concentration changes of or in the body of said patient or nun-human animal,
wherein the active nucleus is preferably hyperpolarized xenon gas, which can be administered through the breathing air (such as by inhaling), Xe mixture or injection of a Xe-saturated carrier solution,
and/or wherein a disease causing changes in metal concentrations is preferably selected from:
- diseases in which calcium signaling is affected
as in:
neuropsychiatric diseases, such as epilepsy,
stroke,
brain damage,
neurodegenerative diseases,
states of altered neuronal processing, such as sleep, coma, anesthesia, intoxication,
cardiovascular diseases, such as arrhythmias, cardiac ischemia and myocardial infarct,
neuromuscular or muscular diseases,
- diseases in which calcium uptake, storage, utilization or excretion is affected
as in
endocrinological conditions, such as hyper/hypoparathyroidism,
malnutrition and gastrointestinal diseases, such as malabsorption and diarrhea *e*.*g*. due to alcoholism,
bone-related diseases, such as osteoporosis,
kidney diseases and treatment with diuretics,
osteoclastic processes of tumors or infections,
diseases in which calcification in tissues occurs, such as cancers, including breast cancer,
atherosclerotic alterations or inflammatory processes,
due to other medical treatments.


SEQUENCE LISTING

<110>  Klinikum rechts der Isar der Technischen Universität
       München
       Forschungsverbund Berlin e.V.

<120>  Molecular sensor for NMR/MRI based on analyte-dependent spectral
       changes of temporarily encapsulated hyperpolarized 129Xe

<130>  U30729EP

<160>  12

<170>  PatentIn version 3.5

<210>  1
<211>  20
<212>  PRT
<213>  Artificial Sequence

<220>
<223>  RS20 peptide

<400>  1

Arg Arg Lys Trp Gln Lys Thr Gly His Ala Val Arg Ala Ile Gly Arg
1               5                   10                  15

Leu Ser Ser Ser
            20

<210>  2
<211>  26
<212>  PRT
<213>  Artificial Sequence

<220>
<223>  M13 peptide

<400>  2

Lys Arg Arg Trp Lys Lys Asn Phe Ile Ala Val Ser Ala Ala Asn Arg
1               5                   10                  15

Phe Lys Lys Ile Ser Ser Ser Gly Ala Leu
            20                  25

<210>  3
<211>  24
<212>  PRT
<213>  Homo sapiens

<400>  3

Ala Arg Lys Glu Val Ile Arg Asn Lys Ile Arg Ala Ile Gly Lys Met
1               5                   10                  15

Ala Arg Val Phe Ser Val Leu Arg

            20

<210>  4
<211>  149
<212>  PRT
<213>  Homo sapiens

<400>  4

<210>  5
<211>  478
<212>  PRT
<213>  Homo sapiens

<400>  5

Met Ala Thr Ile Thr Cys Thr Arg Phe Thr Glu Glu Tyr Gln Leu Phe
1               5                   10                  15

Glu Glu Leu Gly Lys Gly Ala Phe Ser Val Val Arg Arg Cys Val Lys
            20                  25                  30

Val Leu Ala Gly Gln Glu Tyr Ala Ala Lys Ile Ile Asn Thr Lys Lys
        35                  40                  45

Leu Ser Ala Arg Asp His Gln Lys Leu Glu Arg Glu Ala Arg Ile Cys
    50                  55                  60

Arg Leu Leu Lys His Pro Asn Ile Val Arg Leu His Asp Ser Ile Ser
65                  70                  75                  80

Glu Glu Gly His His Tyr Leu Ile Phe Asp Leu Val Thr Gly Gly Glu
                85                  90                  95

Leu Phe Glu Asp Ile Val Ala Arg Glu Tyr Tyr Ser Glu Ala Asp Ala
            100                 105                 110

Ser His Cys Ile Gln Gln Ile Leu Glu Ala Val Leu His Cys His Gln
        115                 120                 125

Met Gly Val Val His Arg Asp Leu Lys Pro Glu Asn Leu Leu Leu Ala
    130                 135                 140

Ser Lys Leu Lys Gly Ala Ala Val Lys Leu Ala Asp Phe Gly Leu Ala
145                 150                 155                 160

Ile Glu Val Glu Gly Glu Gln Gln Ala Trp Phe Gly Phe Ala Gly Thr
                165                 170                 175

Pro Gly Tyr Leu Ser Pro Glu Val Leu Arg Lys Asp Pro Tyr Gly Lys
            180                 185                 190

Pro Val Asp Leu Trp Ala Cys Gly Val Ile Leu Tyr Ile Leu Leu Val
        195                 200                 205

Gly Tyr Pro Pro Phe Trp Asp Glu Asp Gln His Arg Leu Tyr Gln Gln
    210                 215                 220

Ile Lys Ala Gly Ala Tyr Asp Phe Pro Ser Pro Glu Trp Asp Thr Val
225                 230                 235                 240

Thr Pro Glu Ala Lys Asp Leu Ile Asn Lys Met Leu Thr Ile Asn Pro
                245                 250                 255

Ser Lys Arg Ile Thr Ala Ala Glu Ala Leu Lys His Pro Trp Ile Ser
            260                 265                 270

His Arg Ser Thr Val Ala Ser Cys Met His Arg Gln Glu Thr Val Asp

275                 280                 285

Cys Leu Lys Lys Phe Asn Ala Arg Arg Lys Leu Lys Gly Ala Ile Leu
    290                 295                 300

Thr Thr Met Leu Ala Thr Arg Asn Phe Ser Gly Gly Lys Ser Gly Gly
305                 310                 315                 320

Asn Lys Lys Asn Asp Gly Val Lys Glu Ser Ser Glu Ser Thr Asn Thr
                325                 330                 335

Thr Ile Glu Asp Glu Asp Thr Lys Val Arg Lys Gln Glu Ile Ile Lys
            340                 345                 350

Val Thr Glu Gln Leu Ile Glu Ala Ile Ser Asn Gly Asp Phe Glu Ser
        355                 360                 365

Tyr Thr Lys Met Cys Asp Pro Gly Met Thr Ala Phe Glu Pro Glu Ala
    370                 375                 380

Leu Gly Asn Leu Val Glu Gly Leu Asp Phe His Arg Phe Tyr Phe Glu
385                 390                 395                 400

Asn Leu Trp Ser Arg Asn Ser Lys Pro Val His Thr Thr Ile Leu Asn
                405                 410                 415

Pro His Ile His Leu Met Gly Asp Glu Ser Ala Cys Ile Ala Tyr Ile
            420                 425                 430

Arg Ile Thr Gln Tyr Leu Asp Ala Gly Gly Ile Pro Arg Thr Ala Gln
        435                 440                 445

Ser Glu Glu Thr Arg Val Trp His Arg Arg Asp Gly Lys Trp Gln Ile
    450                 455                 460

Val His Phe His Arg Ser Gly Ala Pro Ser Val Leu Pro His
465                 470                 475

<210>  6
<211>  161
<212>  PRT
<213>  Homo sapiens

<400>  6

Met Asp Asp Ile Tyr Lys Ala Ala Val Glu Gln Leu Thr Glu Glu Gln
1               5                   10                  15

Lys Asn Glu Phe Lys Ala Ala Phe Asp Ile Phe Val Leu Gly Ala Glu

20                  25                  30

Asp Gly Cys Ile Ser Thr Lys Glu Leu Gly Lys Val Met Arg Met Leu
        35                  40                  45

Gly Gln Asn Pro Thr Pro Glu Glu Leu Gln Glu Met Ile Asp Glu Val
    50                  55                  60

Asp Glu Asp Gly Ser Gly Thr Val Asp Phe Asp Glu Phe Leu Val Met
65                  70                  75                  80

Met Val Arg Cys Met Lys Asp Asp Ser Lys Gly Lys Ser Glu Glu Glu
                85                  90                  95

Leu Ser Asp Leu Phe Arg Met Phe Asp Lys Asn Ala Asp Gly Tyr Ile
            100                 105                 110

Asp Leu Asp Glu Leu Lys Ile Met Leu Gln Ala Thr Gly Glu Thr Ile
        115                 120                 125

Thr Glu Asp Asp Ile Glu Glu Leu Met Lys Asp Gly Asp Lys Asn Asn
    130                 135                 140

Asp Gly Arg Ile Asp Tyr Asp Glu Phe Leu Glu Phe Met Lys Gly Val
145                 150                 155                 160

Glu

<210>  7
<211>  22
<212>  PRT
<213>  Artificial Sequence

<220>
<223>  Cr-A@K1_RS20_Fluorescein

<400>  7

Gly Arg Arg Lys Trp Gln Lys Thr Gly His Ala Val Arg Ala Ile Gly
1               5                   10                  15

Arg Leu Ser Ser Ser Cys
            20

<210>  8
<211>  22
<212>  PRT
<213>  Artificial Sequence

<220>

<223>  Cr-A@K3_RS20_Fluorescein

<400>  8

Gly Arg Arg Lys Trp Gln Lys Thr Gly His Ala Val Arg Ala Ile Gly
1               5                   10                  15

Arg Leu Ser Ser Ser Cys
            20

<210>  9
<211>  28
<212>  PRT
<213>  Artificial Sequence

<220>
<223>  Cr-A@K1_M13_Fluorescein

<400>  9

Gly Lys Arg Arg Trp Lys Lys Asn Phe Ile Ala Val Ser Ala Ala Asn
1               5                   10                  15

Arg Phe Lys Lys Ile Ser Ser Ser Gly Ala Leu Cys
            20                  25

<210>  10
<211>  22
<212>  PRT
<213>  Artificial Sequence

<220>
<223>  Cr-A@K1_RS20_NEM

<400>  10

Gly Arg Arg Lys Trp Gln Lys Thr Gly His Ala Val Arg Ala Ile Gly
1               5                   10                  15

Arg Leu Ser Ser Ser Cys
            20

<210>  11
<211>  28
<212>  PRT
<213>  Artificial Sequence

<220>
<223>  Cr-A@K1_M13_NEM

<400>  11

Gly Lys Arg Arg Trp Lys Lys Asn Phe Ile Ala Val Ser Ala Ala Asn
1               5                   10                  15

Arg Phe Lys Lys Ile Ser Ser Ser Gly Ala Leu Cys

20                  25


<210>  12
<211>  28
<212>  PRT
<213>  Artificial Sequence

<220>
<223>  CalcA shown in Figure 4B

<400>  12

Gly Cys Gly Ala Arg Lys Glu Val Ile Arg Asn Lys Ile Arg Ala Ile
1               5                   10                  15


Gly Lys Met Ala Arg Val Phe Ser Val Leu Arg Gly
            20                  25